# EUROPEAN PATENT APPLICATION

(11) **EP 4 782 052 A1**
(43) Date of publication of application: **29.07.2026**
(21) Application number: 24868799.8
(22) Date of filing: 19.09.2024
(51) Int. Cl.: A61N 1/04, A61N 1/36, H01B 1/02, H01B 5/14

(54) **NEURAL STIMULATION DEVICE**

(30) Priority: 19.09.2023 KR 20230125069; 19.09.2023 KR 20230125091; 19.09.2023 KR 20230125104
(71) Applicant: LG Innotek Co., Ltd., Seoul 07796 (KR)
(72) Inventor: KEUM, Do Hee, Seoul 07796 (KR)
(74) Representative: DREISS Patentanwälte PartG mbB
(86) International application number: PCT/KR2024/096196
(87) International publication number: WO 2025/063825

(57) **Abstract**

A neural stimulation device according to an embodiment includes a substrate including a first surface and a second surface opposite to the first surface; a first pattern electrode disposed on the first surface; a second pattern electrode disposed on the second surface; a first protective layer disposed on the first pattern electrode; and a second protective layer disposed on the second pattern electrode, wherein the first pattern electrode includes a plurality of branch electrodes, a merge electrode connecting the plurality of branch electrodes, and a protrusion protruding from the branch electrode or the merge electrode, the protrusion includes at least one of a first protrusion connected to the branch electrode and a second protrusion connected to the merge electrode, and a first spacing between a branch electrode adjacent to the first protrusion and the first protrusion is 5% to 45% of a second spacing between the branch electrodes.

## Description

### Technical Field

An embodiment relates to a neural stimulation device.

### Background Art

Many patients are being treated for visual disorders. For example, many patients are treated for visual disorders such as dry eye syndrome, decreased visual acuity, or ocular inflammation.

Recently, with the advancement of technology, neuromodulation has rapidly developed. Accordingly, understanding of the growth and function of neural cells has increased. In addition, it has been discovered that, by accumulated technology, neural regeneration can be activity-controlled by intrinsic characteristics, and in regeneration of peripheral neural tissue, regeneration of neural tissue and other tissues can be accelerated by using minute electrical stimulation.

Accordingly, neural stimulation devices using minute electrical stimulation are being developed. In this regard, various methods for treating visual disorders by delivering electrical stimulation to neural tissue of an eye have been proposed.

Since the neural stimulation device is used in a human body, it includes materials suitable for the human body. Accordingly, a substrate, electrodes, and a protective layer all include materials suitable for the human body.

The neural stimulation device includes a plurality of pattern electrodes. For example, a first pattern electrode may be disposed on a first surface of the substrate, and a second pattern electrode may be disposed on a second surface. In addition, a chip mounting region may be formed on the second surface.

The first pattern electrode and the chip mounting region may overlap. In addition, in order to form the first pattern electrode, a photosensitive film dry film resist may be disposed on the first surface. Accordingly, a step may be formed between the photosensitive film on the first surface overlapping the chip mounting region and the photosensitive film on the first surface not overlapping the chip mounting region.

Accordingly, peeling of the photosensitive film may occur. As a result, a plating layer may be formed even in a space between the first pattern electrodes to form a pattern electrode. Accordingly, a short circuit of the first pattern electrodes may occur.

Accordingly, a neural stimulation device capable of solving the above-described problem is required.

As a technology related to the present invention, Korean Publication Patent No. KR10-2018-0125997 is disclosed.

### Disclosure

### Technical Problem

An embodiment provides a neural stimulation device having improved reliability.

Technical problems to be solved by the proposed embodiments are not limited to the above-mentioned technical problems, and other technical problems not mentioned may be clearly understood by those skilled in the art to which the embodiments proposed from the following descriptions belong.

### Technical Solution

A neural stimulation device according to an embodiment comprises a substrate including a first surface and a second surface opposite to the first surface; a first pattern electrode disposed on the first surface; a second pattern electrode disposed on the second surface; a first protective layer disposed on the first pattern electrode; and a second protective layer disposed on the second pattern electrode, wherein the first pattern electrode includes a plurality of branch electrodes, a merge electrode connecting the plurality of branch electrodes, and a protrusion protruding from the branch electrode or the merge electrode, wherein the protrusion includes at least one of a first protrusion connected to the branch electrode and a second protrusion connected to the merge electrode, and wherein a first spacing between a branch electrode adjacent to the first protrusion and the first protrusion is 5% to 45% of a second spacing between the branch electrodes.

In addition, the substrate includes a mounting region in which an electronic component is disposed, and the mounting region does not overlap the first pattern electrode in a thickness direction of the substrate.

In addition, a line width of the branch electrode is 250 µm to 330 µm, a length of the protrusion is 10 µm to 50 µm, and the second spacing is 60 µm to 110 µm.

In addition, sizes of the first protrusion and the second protrusion are different.

In addition, the substrate includes a mounting region in which an electronic component is disposed, the mounting region does not overlap the branch electrode in a thickness direction of the substrate, and the mounting region overlaps the merge electrode in the thickness direction of the substrate.

In addition, a length of the second protrusion is longer than a length of the first protrusion.

In addition, a length of the second protrusion is 50% to 100% of the second spacing.

In addition, the first protrusion overlaps the second protective layer in a thickness direction of the substrate, and the second protrusion does not overlap the second protective layer in the thickness direction of the substrate.

In addition, the second pattern electrode is disposed in a closed-loop shape.

In addition, the neural stimulation device further comprises a plurality of vias penetrating the first surface and the second surface of the substrate, wherein the first pattern electrode and the second pattern electrode are connected by the vias, the first pattern electrode includes a first metal layer and a second metal layer, the first metal layer and the second metal layer are disposed on an inner surface and a lower surface of the via, and the first protective layer is disposed inside the via.

In addition, a thickness of the second pattern electrode is smaller than a thickness of the first pattern electrode.

In addition, the second pattern electrode includes a plurality of pad parts, a bonding layer is disposed on the pad parts, and the electronic component is disposed on the bonding layer.

A neural stimulation device according to another embodiment includes: a substrate including a first surface and a second surface opposite to the first surface; a first pattern electrode disposed on the first surface; a second pattern electrode disposed on the second surface; a first protective layer disposed on the first pattern electrode; and a second protective layer disposed on the second pattern electrode, wherein the substrate includes a plurality of vias, the first pattern electrode and the second pattern electrode are connected by the vias, the first pattern electrode includes a metal layer including a first metal layer and a second metal layer, the metal layer is disposed on an inner surface and a lower surface of each via, the first protective layer is disposed inside each via, wherein the substrate, the first protective layer, and the second protective layer include a liquid crystal polymer material, and a melting point of the first protective layer is lower than a melting point of the substrate.

A neural stimulation device according to another embodiment includes: a substrate including a first surface and a second surface opposite to the first surface; a first pattern electrode disposed on the first surface; a second pattern electrode disposed on the second surface; a bonding layer disposed on the first pattern electrode; a first protective layer disposed on the bonding layer; and a second protective layer disposed on the second pattern electrode, wherein the substrate includes a plurality of vias, the first pattern electrode and the second pattern electrode are connected by the vias, the first pattern electrode includes a metal layer including a first metal layer and a second metal layer, the metal layer is disposed on an inner surface and a lower surface of each via, the bonding layer is disposed inside each via, the substrate, wherein the first protective layer, and the second protective layer include a liquid crystal polymer material, and a melting point of the bonding layer is lower than melting points of the substrate, the first protective layer, and the second protective layer.

In addition, the melting point of the first protective layer is lower than the melting point of the second protective layer.

In addition, the melting point of the first protective layer is 200°C to 280°C.

In addition, the melting point of the second protective layer is lower than the melting point of the substrate.

In addition, the melting points of the first protective layer and the second protective layer are 200°C to 280°C.

In addition, a water absorption rate of the bonding layer is 0.04% to 0.08%.

In addition, the melting point of the bonding layer is 155°C to 195°C.

In addition, a thickness of the first protective layer is greater than a thickness of the bonding layer.

In addition, the thickness of the first protective layer is greater than a thickness of at least one of the second protective layer and the substrate.

In addition, the first pattern electrode includes a plurality of branch electrodes extending in one direction and a merge electrode connecting the branch electrodes.

In addition, the second pattern electrode is disposed in a closed-loop shape.

In addition, a thickness of the second pattern electrode is smaller than a thickness of the first pattern electrode.

In addition, the second pattern electrode includes a plurality of pad parts, and a bonding layer is disposed on the pad parts.

A neural stimulation device according to another embodiment includes: a substrate including a first surface and a second surface opposite to the first surface; a first pattern electrode disposed on the first surface; a second pattern electrode disposed on the second surface; a first protective layer disposed on the first pattern electrode; a second protective layer disposed on the second pattern electrode; an electronic component disposed on the second pattern electrode; and a molding part surrounding the electronic component, wherein the second protective layer includes a plurality of layers, the second protective layer includes a plurality of protective layers disposed on a side surface of the molding part and a protective layer disposed on an upper surface of the molding part, and the protective layers disposed on the side surface of the molding part have a step.

A neural stimulation device according to another embodiment includes: a substrate including a first surface and a second surface opposite to the first surface; a first pattern electrode disposed on the first surface; a second pattern electrode disposed on the second surface; a first protective layer disposed on the first pattern electrode; a second protective layer disposed on the second pattern electrode; an electronic component disposed on the second pattern electrode; and a molding part surrounding the electronic component, wherein the substrate includes a mounting region in which the electronic component is disposed, the substrate includes a first region overlapping the mounting region and a second region not overlapping the mounting region, and a thickness of the second protective layer on the first region is greater than a thickness of the second protective layer on the second region.

In addition, the second protective layer includes a second-first protective layer to a second-fourth protective layer, the second-first protective layer, the second-second protective layer, and the second-third protective layer are disposed on a side surface of the molding part, the second-first protective layer is disposed on the substrate, the second-second protective layer is disposed on the second-first protective layer, the second-third protective layer is disposed on the second-second protective layer, and a first end of the second-first protective layer protrudes toward the molding part more than a first end of the second-second protective layer and a first end of the second-third protective layer.

In addition, a first end of the second-second protective layer protrudes toward the molding part more than a first end of the second-third protective layer.

In addition, a first end of the second-third protective layer protrudes toward the molding part more than a first end of the second-second protective layer.

In addition, a first end of the second-third protective layer protrudes toward the molding part more than a first end of the second-second protective layer.

In addition, the second protective layer includes a second-first protective layer to a second-fourth protective layer, the second-first protective layer to the second-fourth protective layer are disposed on the first region, and the second-first protective layer is disposed on the second region.

In addition, at least one of a second-second protective layer disposed on the second-first protective layer and a second-third protective layer disposed on the second-second protective layer is further disposed on the second region.

In addition, a sum of thicknesses of the second-first protective layer, the second-second protective layer, and the second-third protective layer is equal to or greater than a thickness of the molding part.

In addition, a thickness of the second-fourth protective layer is smaller than a thickness of at least one of the second-first protective layer, the second-second protective layer, and the second-third protective layer.

In addition, the mounting region does not overlap the first pattern electrode in a thickness direction of the substrate.

In addition, a water absorption rate of the second protective layer is lower than that of the molding part.

In addition, the first pattern electrode includes a plurality of branch electrodes extending in one direction and a merge electrode connecting the branch electrodes, and the second pattern electrode is disposed in a closed-loop shape.

In addition, the substrate includes a plurality of vias, the first pattern electrode and the second pattern electrode are connected by the vias, the first pattern electrode includes a metal layer including a first metal layer and a second metal layer, the metal layer is disposed on an inner surface and a lower surface of each via, and the first protective layer is disposed inside each via.

### Advantageous Effects

A neural stimulation device according to an embodiment includes a first pattern electrode and a second pattern electrode. The first pattern electrode includes branch electrodes and a merge electrode. The first pattern electrode includes a protrusion. The protrusion is connected to at least one electrode of the branch electrodes and the merge electrode.

A size of the protrusion is controlled. In detail, the size of the protrusion is controlled to be less than a spacing between the branch electrodes. Accordingly, it is possible to prevent the branch electrodes from being connected by contact of the protrusions. Accordingly, it is possible to prevent a stimulation signal moving through one branch electrode from moving to another branch electrode. Therefore, driving characteristics of the neural stimulation device are improved.

Strength of the first pattern electrode is improved by the protrusion. Accordingly, it is possible to prevent the first pattern electrode from being damaged by stress generated when the neural stimulation device is bent. In addition, it is possible to prevent the first pattern electrode from being deformed by stress generated when the neural stimulation device is attached to or detached from a face. Therefore, reliability of the neural stimulation device is improved.

In addition, the neural stimulation device according to the embodiment includes a first protective layer and a second protective layer. The first protective layer is disposed while filling an interior of each via.

A melting point of the first protective layer is lower than a melting point of the second protective layer. Accordingly, the first protective layer may be thermocompression-bonded at a low temperature. Therefore, it is possible to prevent a metal layer inside each via from being damaged during a process of forming the first protective layer. In addition, since a temperature of the thermocompression process is lowered, pressure may be increased. Therefore, the first protective layer may be sufficiently disposed inside each via. Accordingly, a void region inside each via may be reduced. Therefore, reliability of the neural stimulation device is improved.

In addition, the neural stimulation device according to the embodiment includes a bonding layer, a first protective layer, and a second protective layer. The bonding layer is disposed while filling the interior of each via. The first protective layer is disposed on the bonding layer.

A melting point of the bonding layer is lower than melting points of the first protective layer and the second protective layer. Accordingly, the bonding layer may be thermocompression-bonded at a low temperature. Therefore, it is possible to prevent the metal layer inside each via from being damaged during a process of forming the bonding layer. In addition, since a temperature of the thermocompression process is lowered, pressure may be increased. Therefore, the bonding layer may be sufficiently disposed inside each via. Accordingly, a void region inside each via may be reduced. Therefore, reliability of the neural stimulation device is improved.

In addition, the bonding layer has a low water absorption rate. Accordingly, it is possible to prevent impurities such as moisture from penetrating into each via. Therefore, reliability of the neural stimulation device is improved.

In addition, the second protective layer is formed of a plurality of layers. The second protective layer includes a second-first protective layer, a second-second protective layer, a second-third protective layer, and a second-fourth protective layer.

The second-first protective layer, the second-second protective layer, and the second-third protective layer are disposed to correspond to a thickness of a molding part protecting the electronic component. In addition, the second-first protective layer, the second-second protective layer, and the second-third protective layer are in contact with the molding part. Accordingly, it is possible to prevent moisture from being introduced into the electronic component.

In addition, even when a crack or peeling of the molding part occurs as the neural stimulation device is bent, the electronic component may be protected by the second protective layer.

In addition, the second-fourth protective layer is disposed on the second-third protective layer and on an upper surface of the molding part. Accordingly, the second protective layer is planarized.

In addition, the second-first protective layer, the second-second protective layer, and the second-third protective layer may be disposed while having a step. Accordingly, a movement path of moisture is lengthened. Therefore, it is possible to prevent moisture from being introduced into the electronic component or into an interior of the neural stimulation device.

In addition, the second surface on which the electronic component is disposed may include a first region and a second region. The first region is a region overlapping a mounting region. The second region is a region not overlapping the mounting region. The second-first protective layer, the second-second protective layer, the second-third protective layer, and the second-fourth protective layer are disposed on the first region. On the second region, at least one protective layer among the second-second protective layer, the second-third protective layer, and the second-fourth protective layer is not disposed.

Accordingly, a thickness of the protective layer on the second region is reduced. Accordingly, reception efficiency of the second pattern electrode may be improved.

### Description of Drawings

FIG. 1 is a view illustrating an example of use of a neural stimulation device according to an embodiment.
FIG. 2 is a view illustrating an assembly including a neural stimulation device according to an embodiment.
FIGS. 3 and 4 are top views of a neural stimulation device according to an embodiment.
FIGS. 5 and 6 are bottom views of a neural stimulation device according to an embodiment.
FIG. 7 is a cross-sectional view taken along A-A' of FIG. 6 according to a first embodiment.
FIG. 8 is a cross-sectional view taken along B-B' of FIG. 6 according to a first embodiment.
FIG. 9 is an enlarged view of region A of FIG. 3 according to the first embodiment.
FIG. 10 is a cross-sectional view taken along D-D' of FIG. 9 according to the first embodiment.
FIG. 11 is another top view of a neural stimulation device according to the first embodiment.
FIG. 12 is a cross-sectional view taken along E-E' of FIG. 11.
FIG. 13 is an enlarged view of region B of FIG. 11.
FIGS. 14 to 19 are views for explaining a method of manufacturing a neural stimulation device according to the first embodiment.
FIGS. 20 to 24 are views for explaining a method of manufacturing a neural stimulation device according to a second embodiment.
FIG. 25 is a view illustrating a photograph of a neural stimulation device according to the second embodiment.
FIG. 26 is a cross-sectional view taken along B-B' of FIG. 6 according to the second embodiment.
FIG. 27 is a view illustrating a photograph of the neural stimulation device according to FIG. 26.
FIG. 28 is a cross-sectional view taken along A-A' of FIG. 6 according to a third embodiment.
FIGS. 29 to 31 are cross-sectional views taken along B-B' of FIG. 6 according to the third embodiment.
FIGS. 32 and 33 are other cross-sectional views taken along A-A' of FIG. 6 according to the third embodiment.
FIGS. 34 to 40 are views for explaining a method of manufacturing a neural stimulation device according to the third embodiment.

### Modes of the Invention

Hereinafter, embodiments of the present disclosure will be described in detail with reference to the accompanying drawings. However, the spirit and scope of the present disclosure is not limited to a part of the embodiments described, and may be implemented in various other forms, and within the spirit and scope of the present disclosure, one or more of the elements of the embodiments may be selectively combined and redisposed.

In addition, unless expressly otherwise defined and described, the terms used in the embodiments of the present disclosure (including technical and scientific terms) may be construed the same meaning as commonly understood by one of ordinary skill in the art to which the present disclosure belongs, and the terms such as those defined in commonly used dictionaries may be interpreted as having a meaning that is consistent with their meaning in the context of the relevant art.

In addition, the terms used in the embodiments of the present disclosure are for describing the embodiments and are not intended to limit the present disclosure. In this specification, the singular forms may also include the plural forms unless specifically stated in the phrase, and may include at least one of all combinations that may be combined in A, B, and C when described in "at least one (or more) of A (and), B, and C".

In addition, in describing the elements of the embodiments of the present disclosure, the terms such as first, second, A, B, (a), and (b) may be used. These terms are only used to distinguish the elements from other elements, and the terms are not limited to the essence, order, or order of the elements.

In addition, when an element is described as being "connected", "coupled", or "contacted" to another element, it may include not only when the element is directly "connected" to, "coupled" to, or "contacted" to other elements, but also when the element is "connected", "coupled", or "contacted" by another element between the element and other elements.

In addition, when described as being formed or disposed "on (over)" or "under (below)" of each element, the "on (over)" or "under (below)" may include not only when two elements are directly connected to each other, but also when one or more other elements are formed or disposed between two elements.

In addition, when expressed as "on (over)" or "under (below)", it may include not only the upper direction but also the lower direction based on one element.

Hereinafter, a neural stimulation device according to an embodiment will be described with reference to the drawings.

Hereinafter, the neural stimulation device according to the embodiment will be described with reference to the drawings.

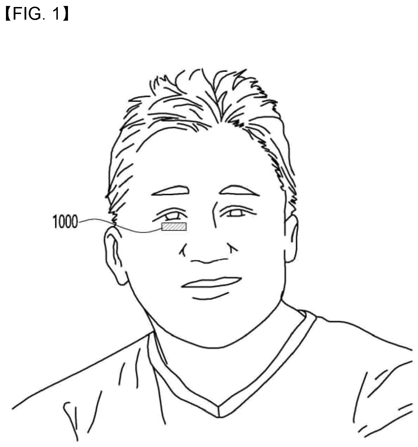

Referring to FIG. 1, the neural stimulation device 1000 is applied to a human body. For example, the neural stimulation device 1000 is attached around an eye of the human body. As an example, the neural stimulation device 1000 may be attached below the eye.

Accordingly, the neural stimulation device 1000 delivers stimulation to neural tissue of the eye. Accordingly, an ocular disorder may be alleviated or treated. For example, dry eye syndrome, ocular inflammation, or decreased visual acuity may be alleviated or treated.

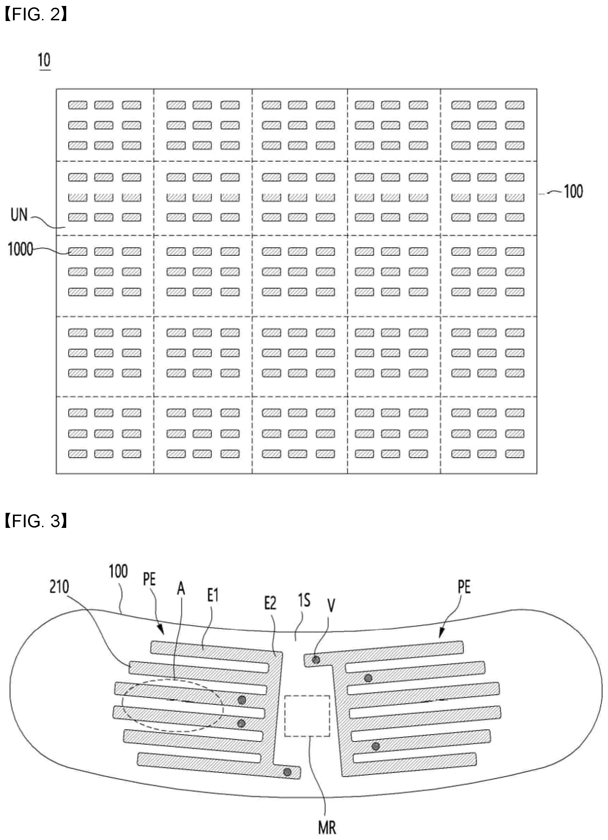

FIG. 2 is a view illustrating an assembly 10 including a plurality of neural stimulation devices 1000. Referring to FIG. 2, the neural stimulation devices 1000 may be manufactured at a panel level. In detail, a plurality of unit regions UN are formed in a large-area substrate 100. Then, each unit region UN may form a plurality of neural stimulation devices 1000. Accordingly, a plurality of neural stimulation devices 1000 may be manufactured in a single process. Therefore, process efficiency is improved.

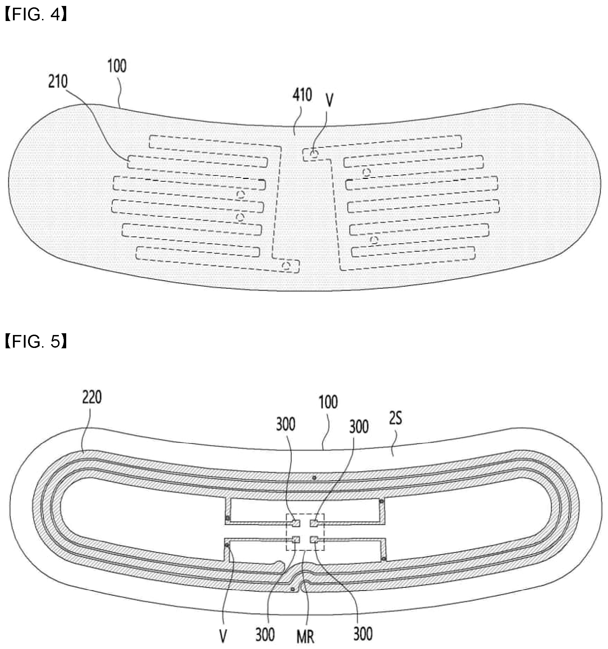

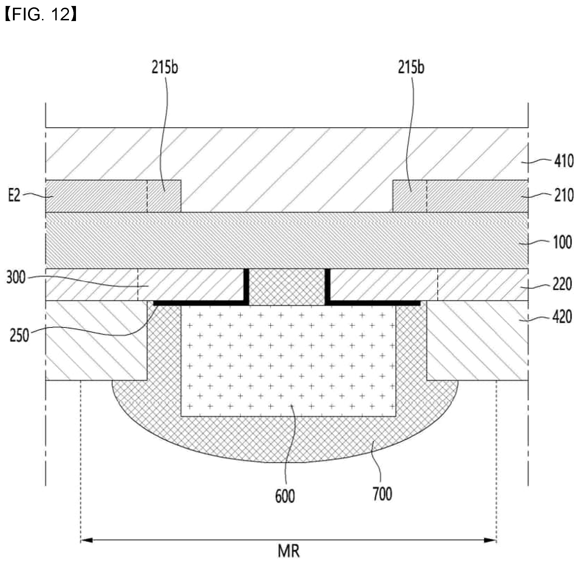

A neural stimulation device 1000 according to an embodiment will be described with reference to FIGS. 3 to 13.

The neural stimulation device 1000 includes a substrate 100, pattern electrodes 210 and 220, and protective layers 410 and 420.

The substrate 100 may include a flexible material. A facial shape differs for each person. Accordingly, as the substrate 100 includes a flexible material, the neural stimulation device 1000 may also be flexible. Accordingly, the neural stimulation device 1000 may be easily bent to match a facial shape of a person. Therefore, neural tissue of the eye may be stimulated through the neural stimulation device 1000.

The substrate 100 may have insulation. Accordingly, the pattern electrodes 210 and 220 disposed on a first surface and a second surface of the substrate 100 may be insulated from each other.

The substrate 100 may include a resin material. For example, the substrate 100 may include a thermoplastic resin. In addition, the substrate 100 may include a material having a low water absorption rate. For example, the water absorption rate of the substrate 100 may be 0.02% to 0.05%.

For example, the substrate 100 may include a liquid crystal polymer material Liquid Crystal Polymer (LCP) or polyamide. The neural stimulation device 1000 contacts skin of a human body. The substrate 100 includes a resin material harmless to the human body. Therefore, when the neural stimulation device is used, side effects that may occur in the human body may be prevented.

The substrate 100 may be formed as a single layer or multiple layers. For example, the substrate 100 may be formed as a single layer. Alternatively, the substrate 100 may be formed as multiple layers. For example, the substrate 100 may be formed by stacking a plurality of the same material. Alternatively, the substrate 100 may be formed by stacking a plurality of materials. The neural stimulation device 1000 may have different sizes depending on the age of use. Since the substrate 100 is formed as a single layer or multiple layers, the neural stimulation device 1000 may be formed to have various thicknesses. Therefore, the neural stimulation device 1000 may be formed in various sizes depending on a use target.

For example, the substrate 100 may have a thickness within a set range. In detail, the thickness of the substrate 100 may be 15 µm to 80 µm, 18 µm to 50 µm, or 20 µm to 30 µm.

If the thickness of the substrate 100 is less than 15 µm, strength of the substrate 100 decreases. An electronic component 600 is disposed on the substrate 100. At this time, heat and pressure are generated. Therefore, the substrate 100 may be damaged. In addition, the substrate 100 may be vulnerable to an impact generated when the substrate 100 is handled. In addition, if the thickness of the substrate 100 exceeds 80 µm, process efficiency may decrease. A plurality of vias are formed in the substrate 100. As the thickness of the substrate 100 increases, a process time for forming the vias and a process time for filling a protective layer into an interior of the vias may increase. In addition, flexible characteristics of the neural stimulation device may decrease. Accordingly, a gap may be formed between the neural stimulation device and skin of the human body, and efficiency of the neural stimulation device may decrease.

The substrate 100 may include at least one via V. For example, the substrate 100 may include a plurality of vias V. The pattern electrodes 210 and 220 are electrically connected through the vias V.

The vias V are formed with a set width. For example, a maximum width of each via V may be 100 µm to 200 µm, 120 µm to 180 µm, or 140 µm to 160 µm.

The width of each via V is related to the thickness of the substrate 100. For example, the width of each via V is proportional to the thickness of the substrate 100.

If the width of each via V is less than 100 µm, the thickness of the substrate 100 may be reduced. Accordingly, as the strength of the substrate 100 decreases, reliability of the neural stimulation device 1000 may decrease. If the width of each via V exceeds 200 µm, the thickness of the substrate 100 may increase. Accordingly, process efficiency and flexible characteristics of the neural stimulation device may decrease.

The substrate includes a first surface 1S and a second surface 2S opposite to the first surface 1S.

The pattern electrodes are disposed on the substrate 100. The pattern electrodes may include a first pattern electrode 210 and a second pattern electrode 220.

The first pattern electrode 210 is disposed on the first surface 1S. The first pattern electrode 210 may include at least one pattern electrode. For example, the first pattern electrode 210 may include a plurality of pattern electrodes PE spaced apart from each other. The plurality of pattern electrodes may be electrically connected through vias.

The first pattern electrode 210 includes a metal. In detail, the first pattern electrode 210 includes a first metal layer 211 and a second metal layer 212.

The first metal layer 211 is disposed on the substrate 100. The first metal layer 211 is in direct contact with the substrate 100. The first metal layer 211 is disposed inside the via V. In detail, the first metal layer 211 is disposed on an inner surface and a lower surface of the via V. The first metal layer 211 includes a material having good adhesion to the substrate 100. For example, the first metal layer 211 may include titanium Ti.

The second metal layer 212 is disposed on the first metal layer 211. The second metal layer 212 is disposed inside the via V. In detail, the second metal layer 212 is disposed on the inner surface and the lower surface of the via V.

The first pattern electrode 210 faces skin of the human body. In detail, neural tissue of the eye may be stimulated through the first pattern electrode 210. As an example, the first pattern electrode 210 may be a stimulation delivery electrode. Therefore, the second metal layer 212 may include a material having good conductivity. In addition, the second metal layer 212 may contact skin of the human body. Accordingly, the second metal layer 212 may include a metal harmless to the human body. For example, the second metal layer 212 may include gold(Au), silver(Ag), or iridium(Ir).

Thicknesses of the first metal layer 211 and the second metal layer 212 are different. In detail, a thickness of the second metal layer 212 is greater than a thickness of the first metal layer 211.

For example, the thickness of the first metal layer 211 may be 10 nm to 40 nm, 20 nm to 38 nm, or 25 nm to 35 nm. If the thickness of the first metal layer 211 is less than 10 nm, bonding strength of the second metal layer 212 may decrease. Accordingly, loss of stimulation delivered to the eye of the human body may occur. If the thickness of the first metal layer 211 exceeds 40 nm, the thickness of the first metal layer 211 may unnecessarily increase. Accordingly, process efficiency may decrease.

The thickness of the second metal layer 212 may be 35 µm to 65 µm, 35 µm to 60 µm, or 45 µm to 55 µm. If the thickness of the second metal layer 212 is less than 35 µm, conductivity of the second metal layer 212 may decrease. Accordingly, intensity of stimulation delivered to the eye of the human body may decrease. If the thickness of the second metal layer 212 exceeds 65 µm, process cost may increase. In addition, the thickness of the second metal layer 212 may unnecessarily increase. Accordingly, process efficiency may decrease.

The first pattern electrode 210 may be formed in various shapes. For example, the first pattern electrode 210 may include a plurality of branch electrodes E1 extending in one direction. The branch electrodes E1 may be spaced apart from each other. In addition, the branch electrodes E1 may be connected by a merge electrode E2.

A stimulation delivery area of the first pattern electrode 210 may be increased by the branch electrodes. In addition, stimulation delivered by the first pattern electrode 210 may become uniform depending on position. That is, since stimulation is delivered by the plurality of branch electrodes, it is possible to prevent stimulation from being concentrated in one region. Therefore, stimulation delivery efficiency of the neural stimulation device may increase.

In addition, stimulation may occur only in some branch electrodes among the branch electrodes. Accordingly, a position of stimulation to be delivered may be controlled according to a user's demand.

The second pattern electrode 220 is disposed on the second surface. The first pattern electrode 210 and the second pattern electrode 220 are electrically connected through the via V.

The second pattern electrode 220 may be formed in a closed-loop shape. For example, the second pattern electrode 220 may be formed in a closed-loop shape wound one or more times.

The second pattern electrode 220 may receive electromagnetic energy from an external wireless device. In addition, the second pattern electrode 220 may generate power required for the electronic component 600 using the electromagnetic energy. As an example, the second pattern electrode 220 may be an antenna electrode.

The second pattern electrode 220 includes a metal. For example, the second pattern electrode 220 may include copper(Cu).

The second pattern electrode 220 overlapping the via V contacts the first metal layer 211 inside the via V. Accordingly, the first pattern electrode 210 and the second pattern electrode 220 are electrically connected.

Thicknesses of the first pattern electrode 210 and the second pattern electrode 220 are different. In detail, a thickness of the second pattern electrode 220 may be smaller than a thickness of the first pattern electrode 210.

For example, the thickness of the second pattern electrode 220 may be 7 µm to 20 µm, 9 µm to 16 µm, or 11 µm to 13 µm. If the thickness of the second pattern electrode 220 is less than 7 µm, conductivity of the second pattern electrode 220 may decrease. Accordingly, reception efficiency of electromagnetic energy through the second pattern electrode may decrease. If the thickness of the second pattern electrode 220 exceeds 20 µm, process cost may increase. In addition, the thickness of the second pattern electrode 220 may unnecessarily increase. Accordingly, process efficiency and flexible characteristics may decrease.

The second pattern electrode 220 includes a plurality of pad parts 300. The electronic component 600 is disposed on the second surface 2S. The electronic component 600 is connected to the second pattern electrode 220 by the pad parts 300.

The substrate 100 includes a mounting region MR. The electronic component 600 is disposed on the mounting region MR. The electronic component 600 is disposed on the second surface 2S.

The electronic component 600 is disposed using heat and pressure. Since the mounting region MR and the first pattern electrode 210 do not overlap, it is possible to prevent the first pattern electrode 210 from being damaged when the electronic component 600 is mounted.

A bonding layer 250 may be disposed on the pad parts 300. The bonding layer 250 may be formed by plating a metal layer on the pad parts 300. The bonding layer 250 may include a first layer on the pad parts 300 and a second layer on the first layer. The first layer may include nickel Ni. The first layer may be disposed to have a thickness of 0.5 µm to 1.5 µm. The second layer may include gold, platinum, or iridium. The second layer may be disposed to have a thickness of 0.03 µm to 0.08 µm.

A solder is disposed between the bonding layer 250 and the electronic component 600, and the electronic component 600 and the second pattern electrode 220 may be connected by the solder. Therefore, electromagnetic energy received by the second pattern electrode 220 may be delivered to the electronic component 600.

A protective layer is disposed on the substrate 100. The protective layer includes a first protective layer 410 and a second protective layer 420. The first protective layer 410 is disposed on the first surface 1S. The first protective layer 410 surrounds the first pattern electrode 210. In addition, the first protective layer 410 is disposed inside the via V. The via V is filled by the first protective layer 410.

The second protective layer 420 is disposed on the second surface 2S. The second protective layer 420 surrounds the second pattern electrode 220. The second protective layer 420 is not disposed on the mounting region MR. Accordingly, the pad parts 300 are exposed to the outside. Therefore, the electronic component 600 may be connected to the pad parts 300.

At least one of the first protective layer 410 and the second protective layer 420 may include a resin material. For example, at least one of the first protective layer 410 and the second protective layer 420 may include a thermoplastic resin. In addition, at least one of the first protective layer 410 and the second protective layer 420 may include a material having a low water absorption rate. For example, the water absorption rate of at least one of the first protective layer 410 and the second protective layer 420 may be 0.02% to 0.05%.

For example, at least one of the first protective layer 410 and the second protective layer 420 may include a liquid crystal polymer material Liquid Crystal Polymer, (LCP) or polyamide. The neural stimulation device 1000 contacts skin of the human body. The protective layers 410 and 420 include a resin material harmless to the human body. Therefore, when the neural stimulation device is used, side effects that may occur in the human body may be prevented.

For example, the first protective layer 410 and the second protective layer 420 may include the same material as the substrate 100. As an example, the substrate 100, the first protective layer 410, and the second protective layer 420 may include a liquid crystal polymer material Liquid Crystal Polymer (LCP).

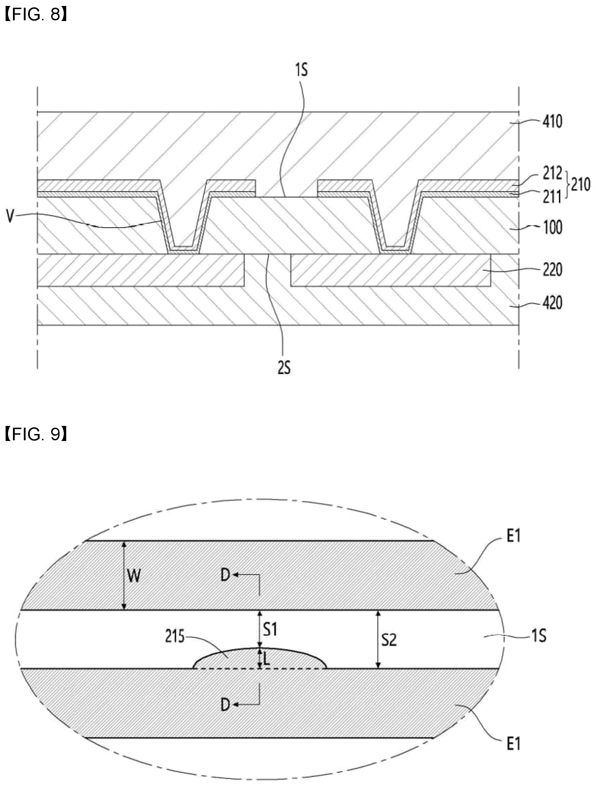

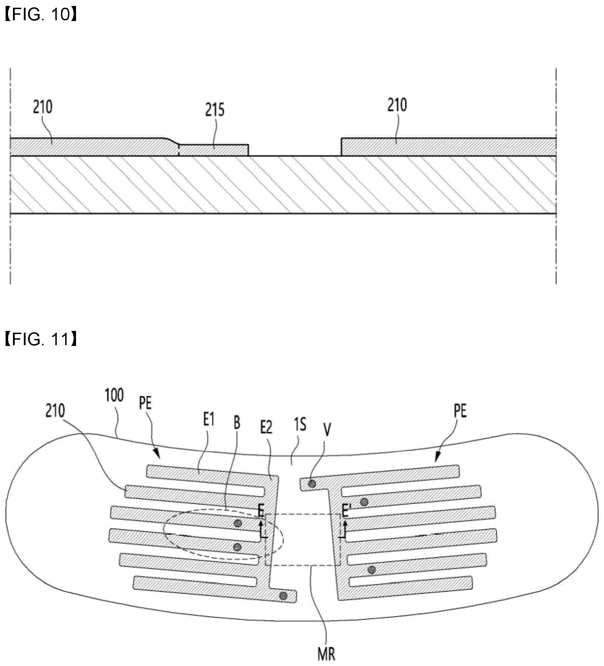

Referring to FIGS. 9 and 10, the first pattern electrode 210 may include a protrusion 215. In detail, the first pattern electrode 210 may include at least one protrusion 215. The protrusion 215 may extend from a side surface of one first pattern electrode 210 toward a side surface of another first pattern electrode 210.

A thickness of the protrusion 215 and a thickness of the first pattern electrode 210 may be different. In detail, the thickness of the protrusion 215 may be smaller than the thickness of the first pattern electrode 210.

The protrusion 215 may be formed during a process of forming the first pattern electrode 210. The first pattern electrode 210 is formed after the second pattern electrode 220 is formed. For example, a process of forming the first pattern electrode 210 may be as follows.

First, the first metal layer 211 and the second metal layer 212 are disposed on the first surface 1S. For example, the first metal layer 211 and the second metal layer 212 are formed by a sputtering process.

Thereafter, a photosensitive material is disposed on the second metal layer 212. Thereafter, a photosensitive pattern is formed through an exposure process. Thereafter, the second metal layer 212 is electrolytically plated. Thereafter, the photosensitive pattern and the first metal layer 211 under the photosensitive pattern are etched.

At this time, adhesion of the photosensitive material may differ depending on a region. Accordingly, after the photosensitive pattern is formed, a portion of the photosensitive pattern may peel off. Accordingly, the second metal layer 212 may be electrolytically plated in an undesired region. Thus, the protrusion 215 may be formed. In addition, since the photosensitive pattern is disposed on a region where the protrusion 215 is formed, the thickness of the protrusion 215 may be smaller than the thickness of the first pattern electrode 210.

Strength of the first pattern electrode 210 may be improved by the protrusion 215. For example, stress generated when the neural stimulation device is bent is distributed in a direction of the protrusion 215, thereby preventing a crack of the branch electrode E1. In addition, when the neural stimulation device is attached to or detached from an eye, deformation of the first pattern electrode 210 may be prevented by the protrusion 215.

However, when the size of the protrusion 215 increases, the protrusion 215 may contact an adjacent pattern electrode PE. Alternatively, protrusions of adjacent pattern electrodes PE may contact each other. Therefore, adjacent branch electrodes E1 may be connected. Accordingly, a large stimulation may occur in one branch electrode. Alternatively, when stimulation is divided among a plurality of branch electrodes to deliver stimulation only to a desired position, stimulation may be delivered to an undesired position.

Therefore, the size of the protrusion 215 may be controlled. In detail, the first pattern electrode 210 includes a first spacing S1 and a second spacing S2. The first spacing S1 is a spacing between a branch electrode E1 adjacent to the protrusion and the protrusion 215. The second spacing S2 is a spacing between adjacent branch electrodes.

The first spacing S1 may be less than 50% of the second spacing S2. In detail, the first spacing S1 may be 5% to 45%, 10% to 43%, or 15% to 40% of the second spacing S2.

For example, a line width W of the branch electrode E1 may be 250 µm to 330 µm. In addition, a length L of the protrusion 215 may be 10 µm to 50 µm. In addition, the second spacing S2 may be 60 µm to 110 µm.

If the first spacing S1 is 50% or more of the second spacing S2, protrusions of branch electrodes may contact each other. Accordingly, adjacent branch electrodes may be connected, thereby causing defects.

If the first spacing S1 is less than 5% of the second spacing S2, strength of the branch electrode may decrease. Accordingly, damage or deformation of the first pattern electrode may occur when the neural stimulation device is used.

The first spacing S1 may be controlled by a position of the mounting region MR. The mounting region MR does not overlap the first pattern electrode 210. In detail, the mounting region MR and the first pattern electrode 210 do not overlap in a thickness direction of the substrate 100.

The protrusion 215 occurs due to reduced adhesion of the photosensitive material. When the mounting region MR overlaps the first pattern electrode 210, a step may be formed in the photosensitive material. The second protective layer 420 is not disposed in the mounting region MR. Therefore, a step is formed on the second surface 2S by the second protective layer 420. Accordingly, adhesion of the photosensitive material disposed in a region corresponding to the mounting region MR may become smaller than adhesion of the photosensitive material disposed in another region due to the step.

Therefore, the mounting region MR does not overlap the first pattern electrode 210. Accordingly, it is possible to prevent adhesion of the photosensitive material from decreasing in a region where the first pattern electrode 210 is disposed. Thus, it is possible to prevent the size of the protrusion 215 from increasing.

Alternatively, the first spacing S1 may be controlled by a manufacturing process of the neural stimulation device. The mounting region MR may overlap the first pattern electrode 210. A temporary bonding material may be disposed on the second protective layer 420. Accordingly, the step caused by the second protective layer 420 may be removed. Thus, it is possible to prevent the size of the protrusion 215 from increasing.

Referring to FIGS. 11 to 13, the protrusion may include a first protrusion 215a and a second protrusion 215b. The first protrusion 215a is connected to the branch electrode E1. At least one first protrusion 215a may be connected to the branch electrode E1. The first protrusion 215a may overlap the second protective layer 420 in a thickness direction of the substrate 100.

The second protrusion 215b is connected to the merge electrode E2. At least one second protrusion 215b may be connected to the merge electrode E2. The second protrusion 215b extends toward an adjacent merge electrode.

A partial region of the merge electrode E2 may overlap the second protective layer 420 in the thickness direction of the substrate 100. In addition, the second protrusion 215b may not overlap the second protective layer 420 in the thickness direction of the substrate 100.

The mounting region MR may overlap the first pattern electrode 210. In detail, the mounting region MR does not overlap the branch electrode E1. In addition, the mounting region MR overlaps the merge electrode E2. In detail, the mounting region MR overlaps a partial region of the merge electrode E2.

Accordingly, sizes of the first protrusion 215a and the second protrusion 215b may be change. In detail, the second protrusion 215b may be larger than the first protrusion 215a. In detail, a length L2 of the second protrusion 215b may be longer than a length L1 of the first protrusion 215a.

The size of the first protrusion 215a may be controlled. In detail, the size of the first protrusion 215a may be controlled in a same manner as the size of the protrusion 215 described above.

The size of the second protrusion 215b may be controlled. In detail, the length L2 of the second protrusion 215b may be 50% or more of the second spacing S2. In detail, the length L2 of the second protrusion 215b may be 50% to 100%, 60% to 90%, or 70% to 80% of the second spacing S2.

For example, a line width of the branch electrode E1 may be 250 µm to 330 µm. In addition, a length L of the protrusion 215 may be 10 µm to 50 µm. In addition, the second spacing S2 may be 60 µm to 110 µm.

The size of the second protrusion 215b may be controlled by the position of the mounting region MR. In detail, the size of the second protrusion 215b may be controlled by a size of an overlapping region between the second protrusion 215b and the mounting region MR. In detail, as the size of the overlapping region increases, the second protrusion 215b increases, and as the size of the overlapping region decreases, the second protrusion 215b decreases.

Since the mounting region overlaps a partial region of the merge electrode, it is possible to prevent a spacing between pattern electrodes of the first pattern electrode from being widened to form the mounting region. Accordingly, a size of the neural stimulation device may be reduced.

In addition, strength of the merge electrode may be improved by the second protrusion. Accordingly, when the neural stimulation device is bent, damage of the merge electrode may be prevented. In addition, when the neural stimulation device is attached or detached, deformation of the merge electrode may be prevented. Therefore, reliability of the neural stimulation device is improved.

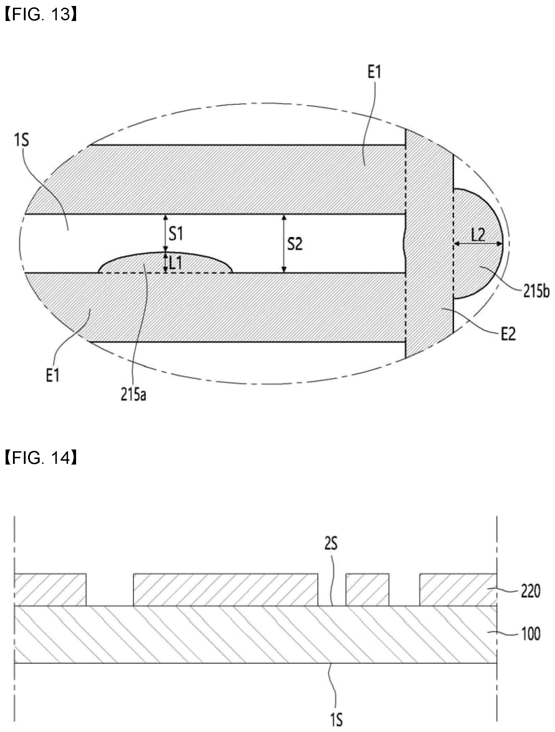

Hereinafter, a method of manufacturing the neural stimulation device according to the embodiment will be described with reference to FIGS. 14 to 19.

Referring to FIG. 14, the substrate 100 is prepared. The substrate 100 includes the resin material described above. A metal layer is disposed on the substrate 100. In detail, copper is disposed on the second surface 2S. Thereafter, the metal layer is patterned. Accordingly, the second pattern electrode 220 is formed on the second surface 2S.

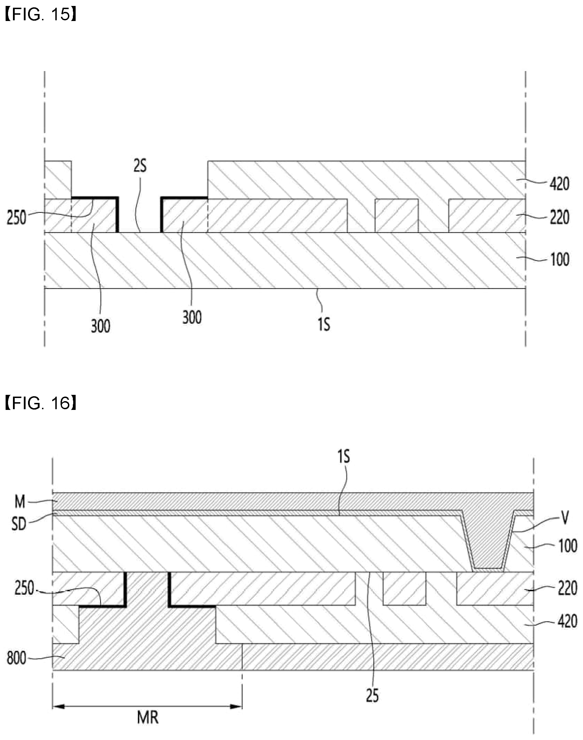

Referring to FIG. 15, the second protective layer 420 is disposed on the second surface 2S. The second protective layer 420 is disposed on the second pattern electrode 220. The second protective layer 420 is disposed while filling a region between the second pattern electrodes 220. The second protective layer 420 is not disposed on the pad parts 300. Accordingly, a step is formed on the second surface 2S by the second protective layer 420. The second pattern electrode 220 is protected by the second protective layer 420.

The pad parts 300 are connected to the electronic component 600. Accordingly, the bonding layer 250 is disposed on the pad parts 300.

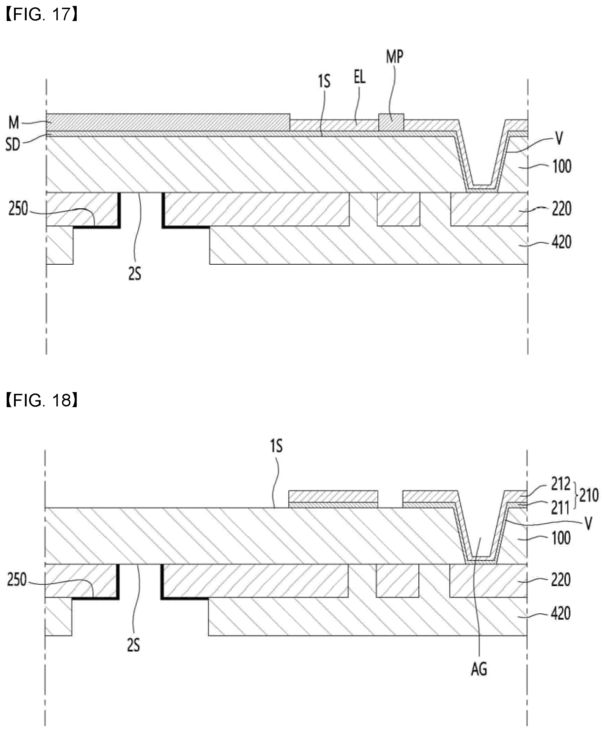

Referring to FIGS. 16 and 17, a seed layer SD is disposed on the first surface 1S. The seed layer includes the first metal layer and the second metal layer described above. A photosensitive material M is disposed on the seed layer SD. Thereafter, the photosensitive material M is patterned. Thereafter, a plating layer EL is formed by electroplating using a seed layer SD between photosensitive patterns MP.

Referring to FIG. 16, a temporary bonding layer 800 may be disposed on the second surface 2S. By the temporary bonding layer 800, the temporary bonding layer 800 may be disposed while filling an interior of the mounting region MR. By the temporary bonding layer 800, the step of the second surface 2S may be removed.

The temporary bonding layer 800 may be selectively disposed. For example, when the first pattern electrode 210 overlaps the mounting region MR, the temporary bonding layer 800 may be disposed. In addition, when the first pattern electrode 210 does not overlap the mounting region MR, the temporary bonding layer 800 may not be disposed.

Referring to FIG. 18, the photosensitive pattern is etched. In addition, the seed layer under the photosensitive pattern is etched. Accordingly, the first pattern electrode 210 is formed on the first surface 1S. In addition, when the temporary bonding layer 800 is disposed, the temporary bonding layer 800 is removed.

The first metal layer 211 and the second metal layer 212 are also disposed inside the via V. In detail, the first metal layer 211 is disposed on the inner surface and the lower surface of the via V. In addition, the second metal layer 212 is disposed on the first metal layer 211.

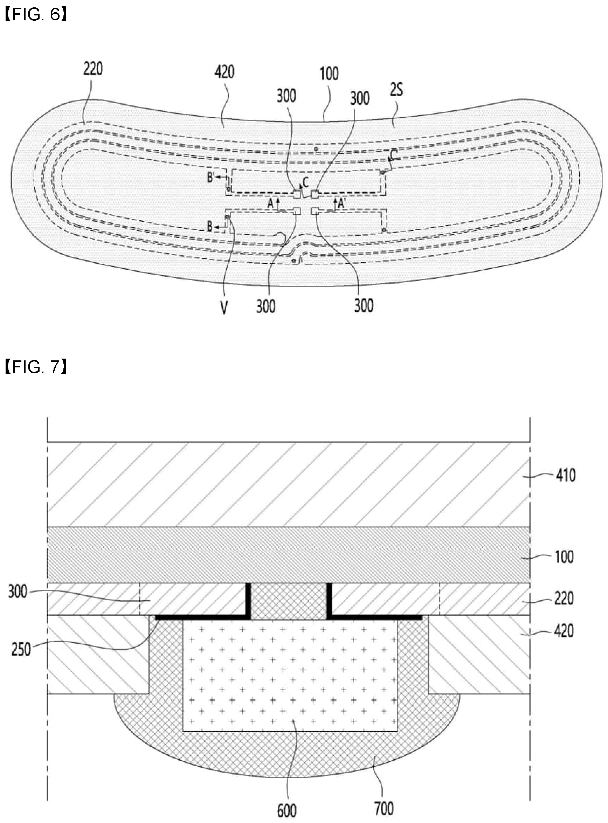

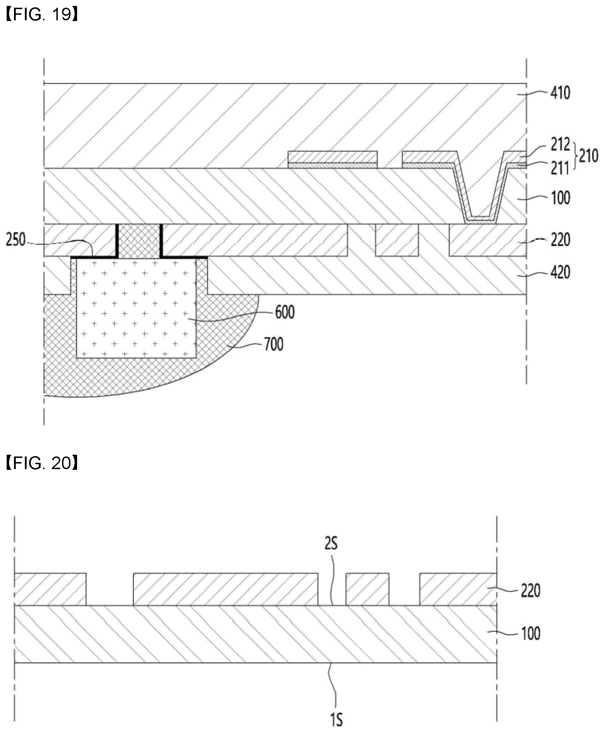

Referring to FIG. 19 (a cross-sectional view taken along C-C' of FIG. 6), the first protective layer 410 is disposed on the first surface 1S. The first protective layer 420 is disposed on the first pattern electrode 210. The first protective layer 410 is disposed while filling a region between the first pattern electrodes 210.

The first protective layer 410 is disposed while filling the interior of the via V. In detail, after aligning the first protective layer 410 on the first surface 1S, the first protective layer 410 may be thermocompression-bonded. Accordingly, the interior of the via V is filled with the first protective layer 410. Therefore, it is possible to prevent impurities such as moisture from being introduced into an interior of the neural stimulation device through the via.

Thereafter, the electronic component 600 is disposed. In detail, after aligning a terminal part of the electronic component 600 and the pad parts 300, the terminal part and the pad parts 300 are bonded through solder. Thereafter, a molding part 700 is disposed on the electronic component 600. The molding part 700 surrounds the electronic component 600. Accordingly, it is possible to prevent impurities such as moisture from being introduced into an interior of the electronic component.

A neural stimulation device according to an embodiment includes a first pattern electrode and a second pattern electrode. The first pattern electrode includes branch electrodes and a merge electrode. The first pattern electrode includes a protrusion. The protrusion is connected to at least one electrode of the branch electrodes and the merge electrode.

A size of the protrusion is controlled. In detail, the size of the protrusion is controlled to be less than a spacing between the branch electrodes. Accordingly, it is possible to prevent the branch electrodes from being connected by contact of the protrusions. Accordingly, it is possible to prevent a stimulation signal moving in one branch electrode from moving to another branch electrode. Therefore, driving characteristics of the neural stimulation device are improved.

Strength of the first pattern electrode is improved by the protrusion. Accordingly, it is possible to prevent the first pattern electrode from being damaged by stress generated when the neural stimulation device is bent. In addition, it is possible to prevent the first pattern electrode from being deformed by stress generated when the neural stimulation device is attached to or detached from a face. Therefore, reliability of the neural stimulation device is improved.

Hereinafter, a method of manufacturing a neural stimulation device according to a second embodiment will be described with reference to FIGS. 20 to 24.

Referring to FIG. 20, the substrate 100 is prepared. The substrate 100 includes the resin material described above. A metal layer is disposed on the substrate 100. In detail, copper is disposed on the second surface 2S. Thereafter, the metal layer is patterned. Accordingly, the second pattern electrode 220 is formed on the second surface 2S.

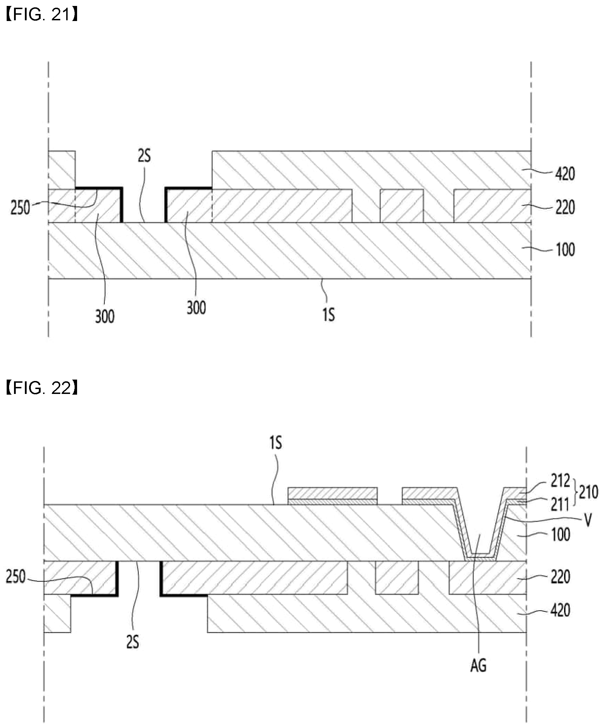

Referring to FIG. 21, the second protective layer 420 is disposed on the second surface 2S. The second protective layer 420 is disposed on the second pattern electrode 220. The second protective layer 420 is disposed while filling a region between the second pattern electrodes 220. The second protective layer 420 is not disposed on the pad parts 300. The second pattern electrode 220 is protected by the second protective layer 420.

The pad parts 300 are connected to the electronic component 600. Accordingly, the bonding layer 250 is disposed on the pad parts 300.

Referring to FIG. 22, a metal layer is disposed on the first surface 1S. In detail, the first metal layer 211 and the second metal layer 212 are disposed on the first surface 1S. Thereafter, the metal layer is patterned. Accordingly, the first pattern electrode 210 is formed on the first surface 1S.

The first metal layer 211 and the second metal layer 212 are also disposed inside the via V. In detail, the first metal layer 211 is disposed on the inner surface and the lower surface of the via V. In addition, the second metal layer 212 is disposed on the first metal layer 211.

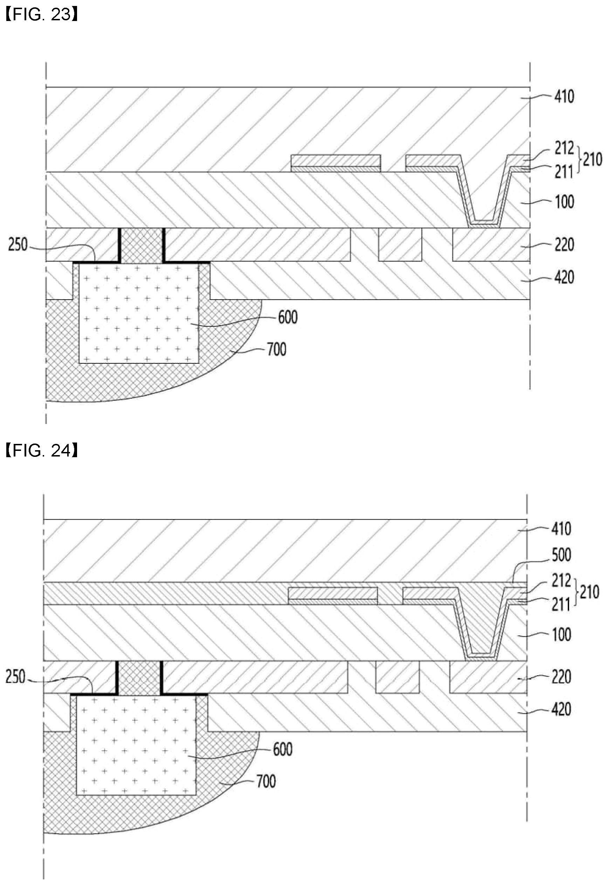

Referring to FIGS. 23 and 24 (a cross-sectional view taken along C-C' of FIG. 6), the first protective layer 410 is disposed on the first surface 1S. The first protective layer 420 is disposed on the first pattern electrode 210. The first protective layer 410 is disposed while filling a region between the first pattern electrodes 210.

The first protective layer 410 is disposed while filling the interior of the via V. In detail, after aligning the first protective layer 410 on the first surface 1S, the first protective layer 410 may be thermocompression-bonded. Accordingly, the interior of the via V is filled with the first protective layer 410. Therefore, it is possible to prevent impurities such as moisture from being introduced into an interior of the neural stimulation device through the via.

Thereafter, the electronic component 600 is disposed. In detail, after aligning a terminal part of the electronic component 600 and the pad parts 300, the terminal and the pad parts 300 are bonded through solder. Thereafter, a molding part 700 is disposed on the electronic component 600. The molding part 700 surrounds the electronic component 600. Accordingly, it is possible to prevent impurities such as moisture from being introduced into an interior of the electronic component.

At this time, the first metal layer 211 and the second metal layer 212 are disposed inside the via V. The first metal layer 211 and the second metal layer 212 are disposed on the inner surface and the lower surface of the via. Accordingly, a void region AG is formed inside the via V. Accordingly, impurities such as moisture may be introduced into an interior of the neural stimulation device through the void region AG. In detail, the neural stimulation device is attached to skin under an eye of a person. Accordingly, sweat or tears of the person may be introduced through the void region.

Accordingly, the first protective layer 410 is disposed on the first pattern electrode 210. Since the first protective layer 410 is disposed while filling the interior of the via V, it is possible to prevent introduction of impurities through the via V.

However, as shown in FIG. 2, the neural stimulation device is manufactured at a panel level. Accordingly, in a via of some neural stimulation devices, the first protective layer 410 may not be disposed inside the via. Alternatively, the first protective layer 410 is not disposed in a sufficient amount inside the via. Accordingly, an area of a void region remaining inside the via V may increase.

In addition, the first protective layer 410 is formed by a thermocompression process. The thermocompression process is performed at heat and pressure of a set magnitude. Accordingly, the thermocompression process is performed at a temperature equal to or higher than a melting point of the first protective layer. When the melting point of the first protective layer is high, the thermocompression process is performed at a high temperature. In addition, the first protective layer is also disposed inside the via. Therefore, compared with a process of forming the second protective layer, pressure may be higher. Accordingly, the metal layer inside the via may be damaged by high temperature and high pressure. Therefore, a crack may occur in the metal layer. Accordingly, reliability of the neural stimulation device may decrease.

The embodiment may solve the above problem by a material of the first protective layer 410 or by addition of a bonding layer.

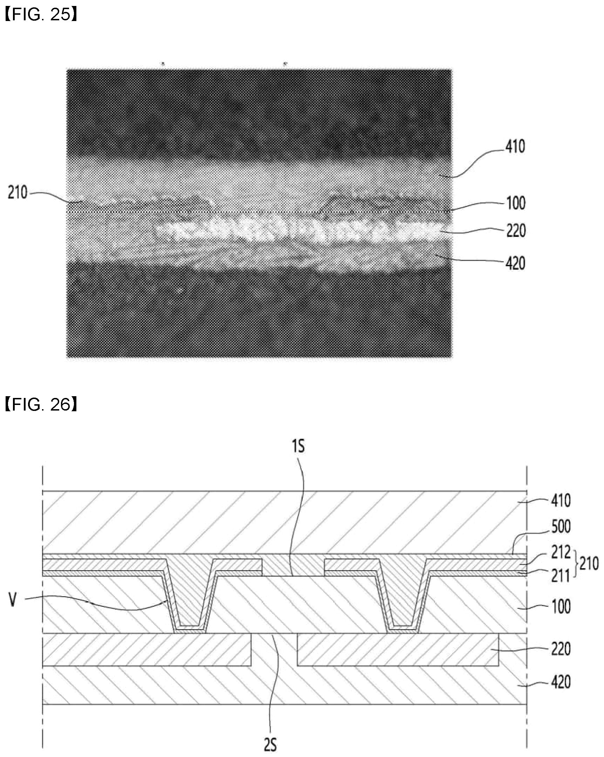

Referring to FIGS. 8 and 25, a protective layer is disposed on the substrate 100. In detail, the first protective layer 410 is disposed on the first surface 1S. The first protective layer 410 surrounds the first pattern electrode 210. In addition, the first protective layer 410 is disposed inside the via V.

The second protective layer 420 is disposed on the second surface 2S. The second protective layer 420 surrounds the second pattern electrode 220.

The first protective layer 410 may include a material different from that of the second protective layer 420. The first protective layer 410 and the second protective layer 420 may have different physical properties. For example, the first protective layer 410 and the second protective layer 420 may include liquid crystal polymer materials having different physical properties. For example, the first protective layer 410 may include a CTF-type liquid crystal polymer material. In addition, the second protective layer 420 may include a CTQ-type liquid crystal polymer material.

The first protective layer 410 may include a material different from that of the substrate 100. The first protective layer 410 and the substrate 100 may have different physical properties.

A melting point of the first protective layer 410 may be different from a melting point of the second protective layer 420. In detail, the melting point of the first protective layer 410 may be lower than the melting point of the second protective layer 420.

In addition, the melting point of the first protective layer 410 may be different from a melting point of the substrate 100. In detail, the melting point of the first protective layer 410 may be lower than the melting point of the substrate 100.

In addition, the melting point of the second protective layer 420 may be the same as or similar to the melting point of the substrate 100. For example, the second protective layer 420 and the substrate 100 may include the same as or similar material. For example, physical properties of the second protective layer 420 and physical properties of the substrate 100 may be the same as or similar.

In detail, the melting point of the first protective layer 410 may be 200°C to 280°C, 230°C to 275°C, or 250°C to 270°C. In addition, the melting point of the second protective layer 410 may be 290°C to 350°C, 295°C to 330°C, or 300°C to 310°C. In addition, the melting point of the substrate 100 may be 290°C to 350°C, 295°C to 330°C, or 300°C to 310°C.

The melting point of the first protective layer 410 is lower than the melting point of the second protective layer 420. Accordingly, a thermocompression process of forming the first protective layer 410 may be performed at a low temperature. Accordingly, it is possible to prevent the metal layer inside the via V from being damaged during the process of forming the first protective layer 410. Therefore, reliability of the neural stimulation device may be improved. However, the embodiment is not limited thereto.

The first protective layer 410 and the second protective layer 420 may include the same as or similar material. In detail, the first protective layer 410 and the second protective layer 420 may have the same as or similar physical properties. Therefore, the melting point of the first protective layer 410 and the melting point of the second protective layer 420 may be the same as or similar.

In addition, the first protective layer 410 and the second protective layer 420 may include materials different from that of the substrate 100. The first protective layer 410 and the second protective layer 420 may have physical properties different from those of the substrate 100.

Therefore, melting points of the first protective layer 410 and the second protective layer 420 may be different from the melting point of the substrate 100. For example, the melting points of the first protective layer 410 and the second protective layer 420 may be lower than the melting point of the substrate 100.

In detail, the melting points of the first protective layer 410 and the second protective layer 420 may be 200°C to 280°C, 230°C to 275°C, or 250°C to 270°C. In addition, the melting point of the substrate 100 may be 290°C to 350°C, 295°C to 330°C, or 300°C to 310°C.

The melting point of the first protective layer 410 is lower than the melting point of the substrate 100. Accordingly, a thermocompression process of forming the first protective layer 410 may be performed at a low temperature. Accordingly, it is possible to prevent the metal layer inside the via V from being damaged during the process of forming the first protective layer 410. Therefore, reliability of the neural stimulation device may be improved.

In addition, the first protective layer 410 and the second protective layer 420 include the same as or similar material. Accordingly, the first protective layer 410 and the second protective layer 420 have the same as or similar melting point. Therefore, thermocompression processes of the first protective layer 410 and the second protective layer 420 may be performed simultaneously. Alternatively, the thermocompression processes of the first protective layer 410 and the second protective layer 420 may be performed using the same equipment. Therefore, process efficiency of the neural stimulation device may be improved.

The first protective layer 410 and the second protective layer 420 have a set thickness. A thickness of the first protective layer 410 may be greater than a thickness of the substrate 100. In addition, the thickness of the first protective layer 410 may be greater than a thickness of the second protective layer 420.

For example, the thickness of the first protective layer 410 may be 35 µm to 100 µm, 40 µm to 90 µm, or 45 µm to 80 µm. The thickness of the first protective layer 410 is defined as a distance from the first surface 1S to an upper surface of the first protective layer 410.

If the thickness of the first protective layer 410 is less than 35 µm, the first protective layer 410 does not sufficiently enter the interior of the via V. Accordingly, as the void region of the via V increases, reliability of the neural stimulation device decreases. If the thickness of the first protective layer 410 exceeds 100 µm, the thickness of the neural stimulation device increases, and thus it may become inconvenient for a user to use the neural stimulation device. For example, when the neural stimulation device is attached below the eye, inconvenience may occur in that the neural stimulation device enters a field of view.

A thickness of the second protective layer 420 may be the same as or similar to the thickness of the substrate 100. In detail, the thickness of the second protective layer 420 may be 15 µm to 80 µm, 18 µm to 50 µm, or 20 µm to 30 µm.

If the thickness of the second protective layer 420 is less than 15 µm, the second pattern electrode 220 is not sufficiently protected by the second protective layer 420. In addition, if the thickness of the second protective layer 420 exceeds 80 µm, reception efficiency of the second pattern electrode 220 may decrease. In detail, reception efficiency of electromagnetic energy delivered from a wireless device may decrease due to the thickness of the second protective layer 420.

The interior of the via V may be filled with another material.

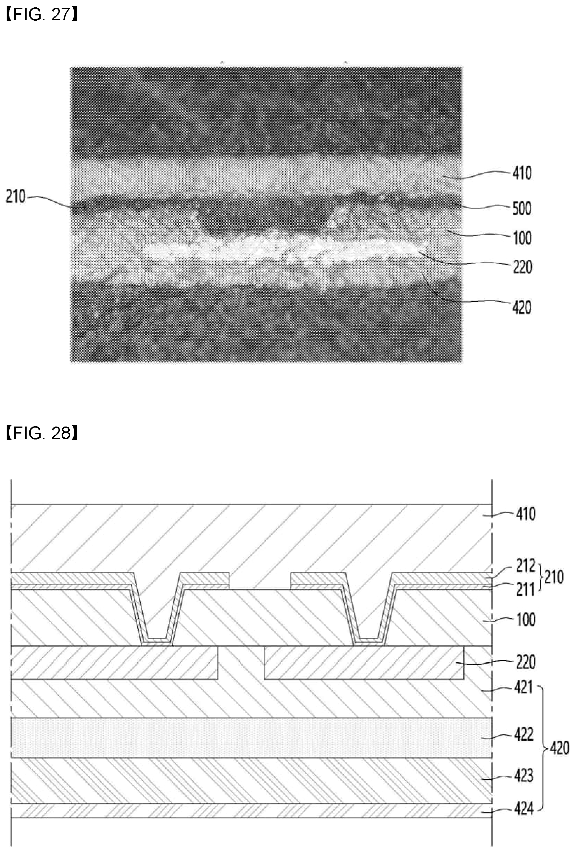

Referring to FIGS. 26 and 27, a bonding layer 500 may be disposed on the first surface 1S. The bonding layer 500 surrounds the first pattern electrode 210. In addition, the bonding layer 500 is disposed inside the via V.

The bonding layer 500 may include a material different from those of the first protective layer 410 and the second protective layer 420. The bonding layer 500 may have physical properties different from those of the first protective layer 410 and the second protective layer 420.

The bonding layer 500 may include a material different from that of the substrate 100. The bonding layer 500 and the substrate 100 may have different physical properties.

The bonding layer 500 may include a resin material. The bonding layer 500 may include a material having a low water absorption rate. For example, the water absorption rate of the bonding layer 500 may be similar to the water absorption rate of the substrate 100. As an example, the water absorption rate of the bonding layer 500 may be 0.04% to 0.08%.

A melting point of the bonding layer 500 may be different from the melting points of the first protective layer 410 and the second protective layer 420. In detail, the melting point of the bonding layer 500 may be lower than the melting points of the first protective layer 410 and the second protective layer 420.

In addition, the melting point of the bonding layer 500 may be different from the melting point of the substrate 100. In detail, the melting point of the bonding layer 500 may be lower than the melting point of the substrate 100.

The melting point of the bonding layer 500 may be 155°C to 195°C, 165°C to 190°C, or 175°C to 185°C. In addition, the melting points of the first protective layer 410 and the second protective layer 420 may be 290°C to 350°C, 295°C to 330°C, or 300°C to 310°C. In addition, the melting point of the substrate 100 may be 290°C to 350°C, 295°C to 330°C, or 300°C to 310°C.

The first protective layer 410 is disposed on the bonding layer 500. The first protective layer 410 and the bonding layer 500 may have different thicknesses. In detail, the thickness of the first protective layer 410 may be greater than the thickness of the bonding layer 500. The bonding layer 500 may be harmful to the human body. Since the thickness of the first protective layer 410 is greater than the thickness of the bonding layer 500, when the first protective layer 410 is damaged, it is possible to prevent the bonding layer 500 from being exposed.

The melting point of the bonding layer 500 is lower than the melting points of the first protective layer 410 and the second protective layer 420. Accordingly, a thermocompression process of forming the bonding layer 500 may be performed at a low temperature. Accordingly, it is possible to prevent the metal layer inside the via V from being damaged during the process of forming the bonding layer 500. Therefore, reliability of the neural stimulation device may be improved.

In addition, the bonding layer 500 has a low water absorption rate. Therefore, it is possible to prevent moisture from penetrating into the interior of the via.

A neural stimulation device according to an embodiment includes a first protective layer and a second protective layer. The first protective layer is disposed while filling the interior of the via.

The melting point of the first protective layer is lower than the melting point of the second protective layer. Accordingly, the first protective layer may be thermocompression-bonded at a low temperature. Therefore, it is possible to prevent the metal layer inside the via from being damaged during a process of forming the first protective layer. In addition, since a temperature of the thermocompression process is lowered, pressure may be increased. Therefore, the first protective layer may be sufficiently disposed inside the via. Therefore, a void region inside the via may be reduced. Therefore, reliability of the neural stimulation device is improved.

In addition, a neural stimulation device according to an embodiment includes a bonding layer, a first protective layer, and a second protective layer. The bonding layer is disposed while filling the interior of the via. The first protective layer is disposed on the bonding layer.

The melting point of the bonding layer is lower than the melting points of the first protective layer and the second protective layer. Accordingly, the bonding layer may be thermocompression-bonded at a low temperature. Therefore, it is possible to prevent the metal layer inside the via from being damaged during a process of forming the bonding layer. In addition, since a temperature of the thermocompression process is lowered, pressure may be increased. Therefore, the bonding layer may be sufficiently disposed inside the via. Therefore, a void region inside the via may be reduced. Therefore, reliability of the neural stimulation device is improved.

In addition, the bonding layer has a low water absorption rate. Therefore, it is possible to prevent impurities such as moisture from penetrating into the interior of the via. Therefore, reliability of the neural stimulation device is improved.

Meanwhile, according to another embodiment, the second protective layer 420 may be provided as a plurality of layers.

That is, referring to FIGS. 28 to 31, a protective layer is disposed on the substrate 100. The protective layer includes a first protective layer 410 and a second protective layer 420. The first protective layer 410 is disposed on the first surface 1S. The first protective layer 410 surrounds the first pattern electrode 210. In addition, the first protective layer 410 is disposed inside the via V. The via V is filled by the first protective layer 410.

The second protective layer 420 is disposed on the second surface 2S. The second protective layer 420 surrounds the second pattern electrode 220.

At least one of the first protective layer 410 and the second protective layer 420 may include a resin material. For example, at least one of the first protective layer 410 and the second protective layer 420 may include a thermoplastic resin. In addition, at least one of the first protective layer 410 and the second protective layer 420 may include a material having a low water absorption rate. For example, the water absorption rate of at least one of the first protective layer 410 and the second protective layer 420 may be 0.02% to 0.05%.

For example, at least one of the first protective layer 410 and the second protective layer 420 may include a liquid crystal polymer material Liquid Crystal Polymer (LCP) or polyamide. The neural stimulation device 1000 contacts skin of the human body. The protective layers 410M and 420 include a resin material harmless to the human body. Therefore, when the neural stimulation device is used, side effects that may occur in the human body may be prevented.

For example, the first protective layer 410 and the second protective layer 420 may include the same material as the substrate 100. As an example, the substrate 100, the first protective layer 410, and the second protective layer 420 may include a liquid crystal polymer material Liquid Crystal Polymer (LCP).

The second protective layer 420 is disposed on the second surface 2S. Accordingly, the second protective layer 420 is disposed on a surface on which the electronic component 600 is disposed.

The second protective layer 420 includes multiple layers. In detail, the second protective layer may include a second-first protective layer 421, a second-second protective layer 422, a second-third protective layer 423, and a second-fourth protective layer 424.

The second-first protective layer 421 is disposed on the second pattern electrode 220. The second-first protective layer 421 surrounds the second pattern electrode 220. The second pattern electrode 220 is protected by the second-first protective layer.

The second-second protective layer 422 is disposed on the second-first protective layer 421. The second-third protective layer 423 is disposed on the second-second protective layer 422.

The second-first protective layer 421, the second-second protective layer 422, and the second-third protective layer 423 contact the molding part 600. In detail, a first end of the second-first protective layer 421, a first end of the second-second protective layer 422, and a first end of the second-third protective layer 423 contact the molding part 600. In detail, the second-first protective layer 421, the second-second protective layer 422, and the second-third protective layer 423 are disposed on a side surface of the molding part 600.

A sum of thicknesses of second protective layers disposed on the side surface of the molding part 600 may be the same as or similar to a thickness of the molding part 600. In detail, the sum of thicknesses of the second protective layers disposed on the side surface of the molding part 600 may be equal to or greater than the thickness of the molding part 600.

For example, a sum of thicknesses of the second-first protective layer 421, the second-second protective layer 422, and the second-third protective layer 423 may be the same as or similar to the thickness of the molding part 600. In detail, the sum of thicknesses of the second-first protective layer 421, the second-second protective layer 422, and the second-third protective layer 423 may be equal to or greater than the thickness of the molding part 600.

Accordingly, the electronic component 600 is surrounded by the molding part 700, the second-first protective layer 421, the second-second protective layer 422, and the second-third protective layer 423.

Therefore, it is possible to prevent moisture from being introduced into the electronic component 600. A crack may occur in the molding part when the neural stimulation device 1000 is bent. Alternatively, the molding part 700 may peel off. Accordingly, moisture may be introduced into the electronic component 600, and reliability of the neural stimulation device may decrease.

However, in the embodiment, the electronic component 600 is protected by the second-first protective layer 421, the second-second protective layer 422, and the second-third protective layer 423. The second-first protective layer 421, the second-second protective layer 422, and the second-third protective layer 423 have a low water absorption rate. In detail, the water absorption rates of the second-first protective layer 421, the second-second protective layer 422, and the second-third protective layer 423 are lower than the water absorption rate of the molding part. In addition, the second-first protective layer 421, the second-second protective layer 422, and the second-third protective layer 423 are flexible. Therefore, when the neural stimulation device is bent, it is possible to prevent peeling or damage of the second-first protective layer 421, the second-second protective layer 422, and the second-third protective layer 423. Accordingly, it is possible to prevent tears or sweat of a person from being introduced into the electronic component or into an interior of the neural stimulation device. Therefore, reliability of the neural stimulation device is improved.

The second-fourth protective layer 424 is disposed on the second-third protective layer 423. The second-fourth protective layer 424 contacts the second-third protective layer 423 and the molding part 700. In detail, the second-fourth protective layer 424 is disposed on an upper surface of the molding part 600. A step between the molding part 700 and the second protective layer 420 may be removed by the second-fourth protective layer 424. Therefore, a bottom surface of the neural stimulation device may be planarized.

A thickness of at least one protective layer among the second-first protective layer 421, the second-second protective layer 422, the second-third protective layer 423, and the second-fourth protective layer 424 may be different from a thickness of another protective layer.

In detail, a thickness of the second-fourth protective layer 424 may be different from thicknesses of the second-first protective layer 421, the second-second protective layer 422, and the second-third protective layer 423. In detail, the thickness of the second-fourth protective layer 424 may be smaller than the thicknesses of the second-first protective layer 421, the second-second protective layer 422, and the second-third protective layer 423.

Since the second-first protective layer 421, the second-second protective layer 422, and the second-third protective layer 423 are disposed by a thickness of the molding part 700, they have a thickness equal to or greater than a set range. Since the second-fourth protective layer 424 is disposed for planarization of the second protective layer, it is disposed to have a minimum thickness. Accordingly, it is possible to prevent an increase in thickness of the neural stimulation device while improving reliability of the neural stimulation device. In addition, since the thickness of the second protective layer 420 is reduced, transmission efficiency of electromagnetic energy delivered to the second pattern electrode 220 may be improved.

For example, the thickness of the second-fourth protective layer 424 may be 10% to 40%, 15% to 35%, or 20% to 30% of a thickness of at least one protective layer among the second-first protective layer 421, the second-second protective layer 422, and the second-third protective layer 423.

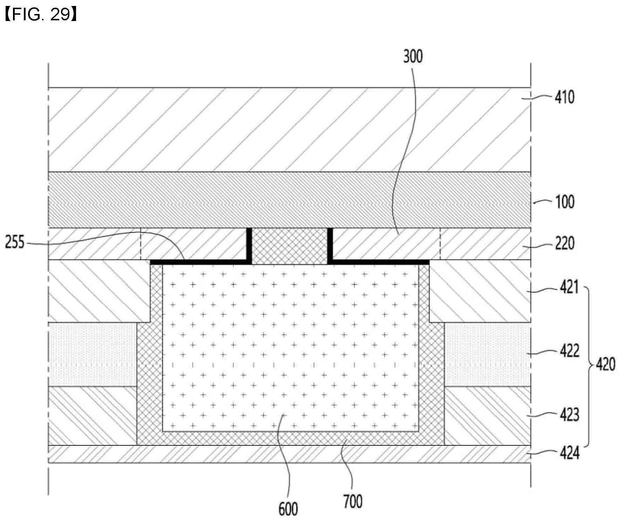

Referring to FIGS. 29 to 31, the second-first protective layer 421, the second-second protective layer 422, and the second-third protective layer 423 may have a step. That is, the protective layers disposed on the side surface of the molding part may have a step.

Referring to FIG. 29, a first end of the second-first protective layer 421 may protrude toward the molding part 700 more than a first end of the second-second protective layer 422 and a first end of the second-third protective layer 423.

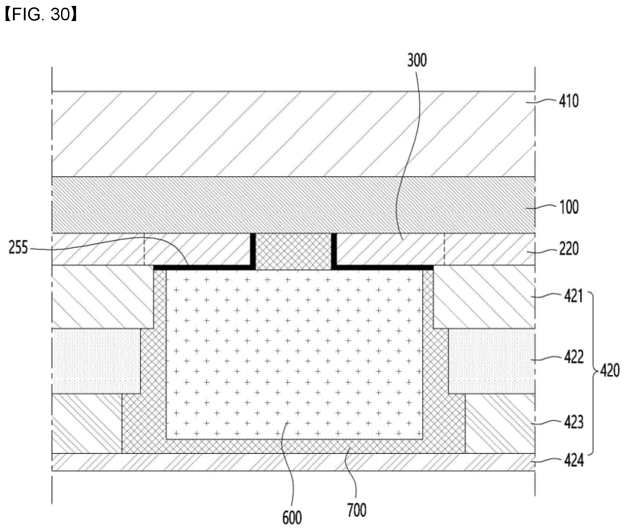

Alternatively, referring to FIG. 30, the first end of the second-first protective layer 421 may protrude toward the molding part 700 more than the first end of the second-second protective layer 422 and the first end of the second-third protective layer 423. In addition, the first end of the second-second protective layer 422 may protrude toward the molding part 700 more than the first end of the second-third protective layer 423.

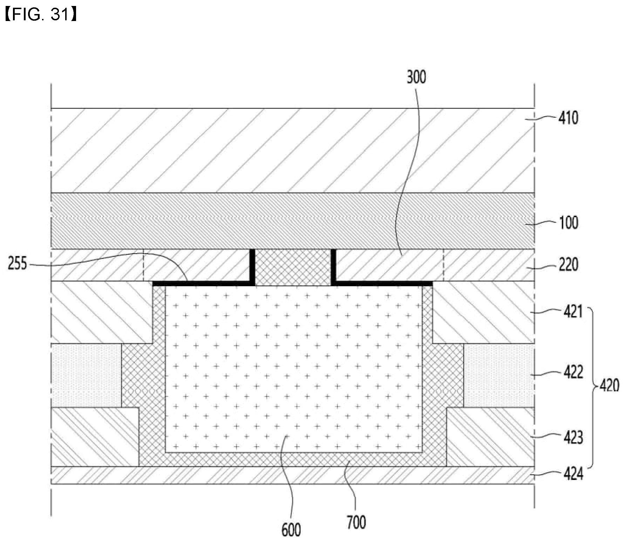

Alternatively, referring to FIG. 31, the first end of the second-first protective layer 421 may protrude toward the molding part 700 more than the first end of the second-second protective layer 422 and the first end of the second-third protective layer 423. In addition, the first end of the second-third protective layer 423 may protrude toward the molding part 700 more than the first end of the second-second protective layer 422.

The step of the second-first protective layer 421, the second-second protective layer 422, and the second-third protective layer 423 is formed by a manufacturing process of the neural stimulation device. In detail, after the second-first protective layer 421 is first disposed, the molding part 700 is disposed. The molding part 700 may be formed in a hemispherical shape. Therefore, the first end of the second-first protective layer 421 may protrude more than the first end of the second-second protective layer 422 and the first end of the second-third protective layer 423.

Thereafter, the second-second protective layer 422 and the second-third protective layer 423 are sequentially disposed. The second-second protective layer 422 and the second-third protective layer 423 are disposed by a thermocompression process. In detail, the second-second protective layer 422 and the second-third protective layer 423 are bonded by heat and pressure.

Depending on heat and pressure applied when the second-second protective layer 422 and the second-third protective layer 423 are disposed, the second-second protective layer 422 and the second-third protective layer 423 may have a step.

For example, when heat and pressure of a thermocompression process of the second-second protective layer 422 are greater than heat and pressure of a thermocompression process of the second-third protective layer 423, the first end of the second-second protective layer 422 may protrude more than the first end of the second-third protective layer 423.

Alternatively, when heat and pressure of a thermocompression process of the second-third protective layer 423 are greater than heat and pressure of a thermocompression process of the second-second protective layer 422, the first end of the second-third protective layer 423 may protrude more than the first end of the second-second protective layer 422.

Since the second-first protective layer 421, the second-second protective layer 422, and the second-third protective layer 423 have a step, reliability of the neural stimulation device may be improved. In detail, a movement path of impurities introduced from the outside increases by the step. Accordingly, it is possible to minimize impurities from being introduced into the electronic component or into the interior of the neural stimulation device.

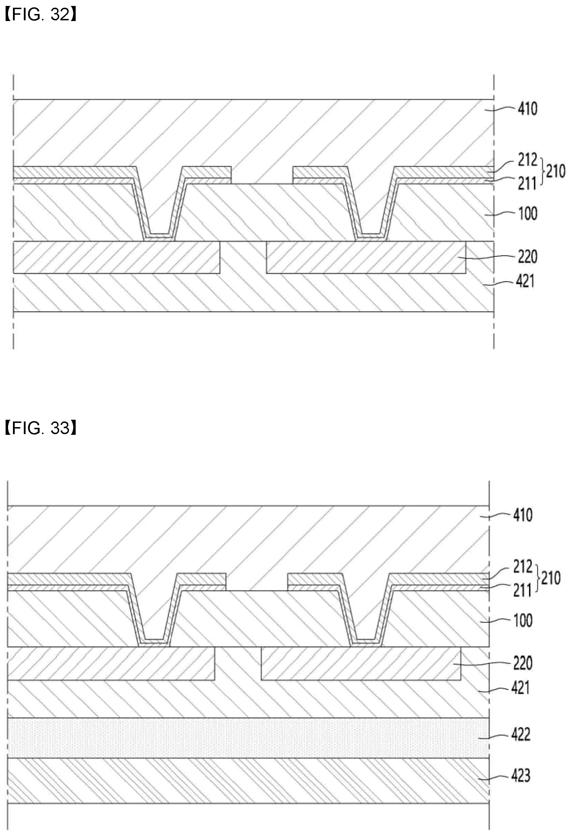

Referring to FIGS. 32 and 33, at least one protective layer among the second-second protective layer 422, the second-third protective layer 423, and the second-fourth protective layer 424 may not be disposed in a region other than the mounting region MR. Accordingly, a thickness of the protective layer disposed in a region overlapping the mounting region MR may be greater than a thickness of the protective layer disposed in a region other than the mounting region MR.

Referring to FIG. 32, the first protective layer 421, the second-second protective layer 422, the second-third protective layer 423, and the second-fourth protective layer 424 are disposed in a region overlapping the mounting region MR. In addition, only the first protective layer 421 may be disposed in a region other than the mounting region MR.

Referring to FIG. 33, the first protective layer 421, the second-second protective layer 422, the second-third protective layer 423, and the second-fourth protective layer 424 are disposed in a region overlapping the mounting region MR. In addition, the first protective layer 421, the second-second protective layer 422, and the second-third protective layer 423 may be disposed in a region other than the mounting region MR. For example, at least one protective layer among the first protective layer 421, the second-second protective layer 422 and the second-third protective layer 423 may be disposed in a region other than the mounting region MR. That is, the second-fourth protective layer 424 may not be disposed in a region other than the mounting region MR.

Therefore, the electronic component 600 is protected by the first protective layer 421, the second-second protective layer 422, the second-third protective layer 423, and the second-fourth protective layer 424. The second pattern electrode 220 is disposed in a region other than the mounting region MR. Since a thickness of the protective layer disposed in a region other than the mounting region MR is reduced, transmission efficiency of electromagnetic energy delivered to the second pattern electrode 220 may be improved. Therefore, driving characteristics of the neural stimulation device are improved.

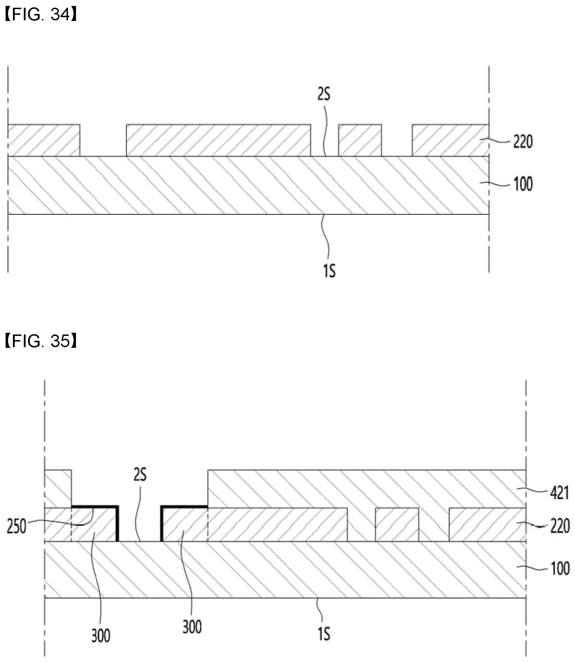

Hereinafter, a method of manufacturing the neural stimulation device according to the embodiment will be described with reference to FIGS. 34 to 40.

Referring to FIG. 34, the substrate 100 is prepared. The substrate 100 includes the resin material described above. A metal layer is disposed on the substrate 100. In detail, copper is disposed on the second surface 2S. Thereafter, the metal layer is patterned. Accordingly, the second pattern electrode 220 is formed on the second surface 2S.

Referring to FIG. 35, the second-first protective layer 421 is disposed on the second surface 2S. The second-first protective layer 421 is disposed on the second pattern electrode 220. The second-first protective layer 421 is disposed while filling a region between the second pattern electrodes 220. The second-first protective layer 421 is not disposed on the pad parts 300. The second pattern electrode 220 is protected by the second-first protective layer 421.

The pad parts 300 are connected to the electronic component 600. Accordingly, the bonding layer 250 is disposed on the pad parts 300.

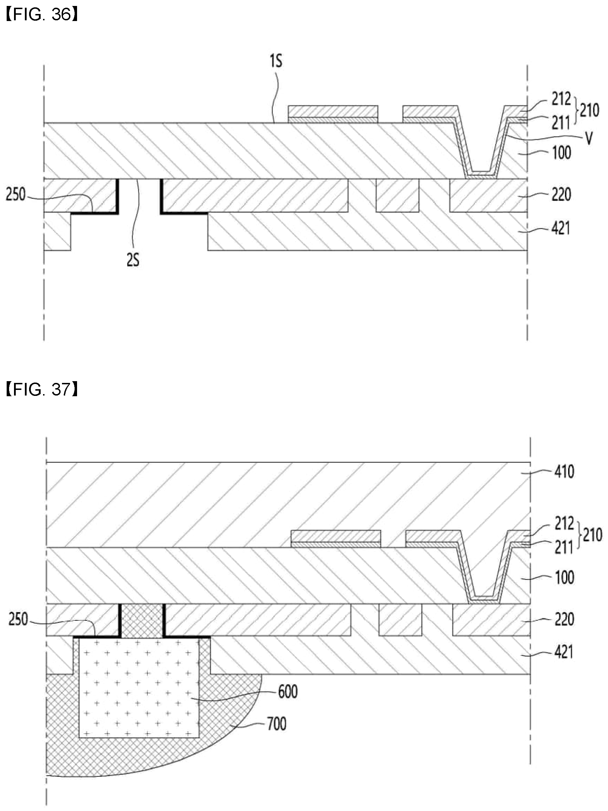

Referring to FIG. 36, a metal layer is disposed on the first surface 1S. In detail, the first metal layer 211 and the second metal layer 212 are disposed on the first surface 1S. Thereafter, the metal layer is patterned. Accordingly, the first pattern electrode 210 is formed on the first surface 1S.

The first metal layer 211 and the second metal layer 212 are also disposed inside the via V. In detail, the first metal layer 211 is disposed on the inner surface and the lower surface of the via V. In addition, the second metal layer 212 is disposed on the first metal layer 211.

Referring to FIG. 37, the first protective layer 410 is disposed on the first surface 1S. The first protective layer 410 is disposed on the first pattern electrode 210. The first protective layer 410 is disposed while filling a region between the first pattern electrodes 210.

The first protective layer 410 is disposed while filling the interior of the via V. In detail, after aligning the first protective layer 410 on the first surface 1S, the first protective layer 410 may be thermocompression-bonded. Accordingly, the interior of the via V is filled with the first protective layer 410. Therefore, it is possible to prevent impurities such as moisture from being introduced into an interior of the neural stimulation device through the via.

Thereafter, the electronic component 600 is disposed. In detail, after aligning a terminal part of the electronic component 600 and the pad parts 300, the terminal and the pad parts 300 are bonded through solder. Thereafter, the molding part 700 is disposed on the electronic component 600. The molding part 700 surrounds the electronic component 600. Accordingly, it is possible to prevent impurities such as moisture from being introduced into an interior of the electronic component.

The molding part 700 includes a curved surface. For example, the molding part 700 may be formed in a hemispherical shape.

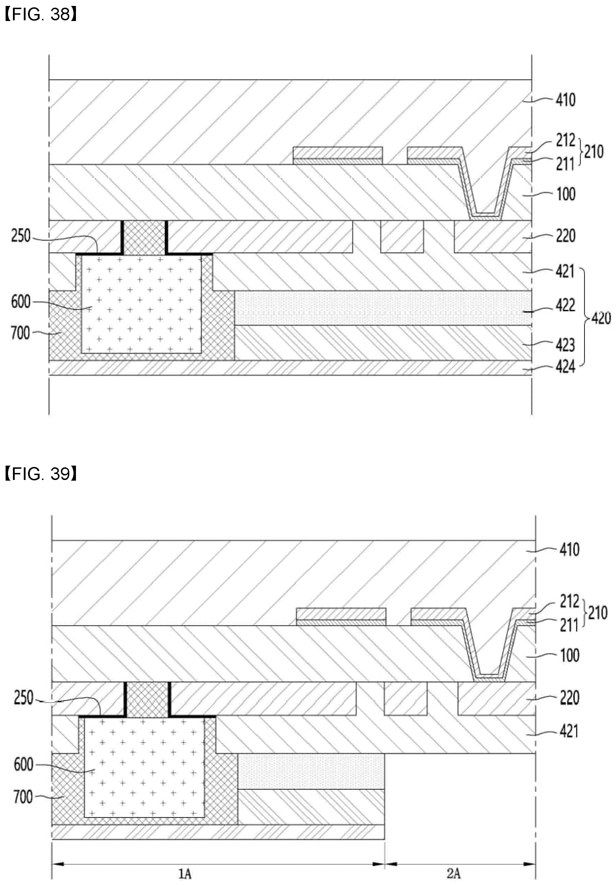

Referring to FIG. 38, the second-second protective layer 422, the second-third protective layer 423, and the second-fourth protective layer 424 are sequentially disposed on the second surface 2S. The second-third protective layer 423 is disposed such that an upper surface thereof has substantially no step with an upper surface of the molding part 700. The second-second protective layer 422, the second-third protective layer 423, and the second-fourth protective layer 424 are disposed by a thermocompression process. Accordingly, they are disposed while pushing the molding part 700 inward. Accordingly, an outer shape of the molding part 700 is changed.

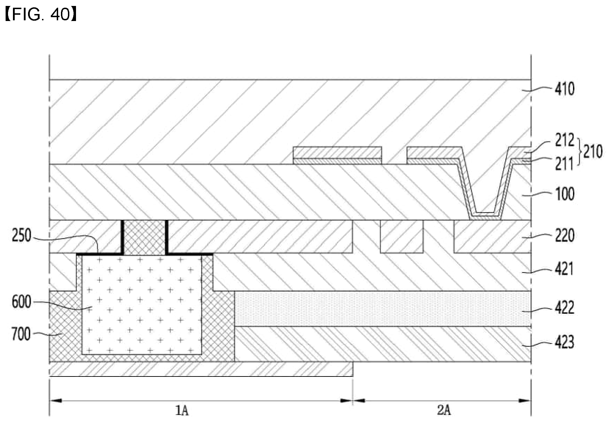

Referring to FIGS. 39 and 40, the second surface 2S may include a first region 1A and a second region 2A.

The first region 1A overlaps the mounting region MR. The second region 2A does not overlap the mounting region MR.

Referring to FIG. 39, the second-first protective layer 421, the second-second protective layer 422, the second-third protective layer 423, and the second-fourth protective layer 424 are disposed on the first region 1A. In addition, only the second-first protective layer 421 is disposed on the second region 2A.

Alternatively, referring to FIG. 40, the second-first protective layer 421, the second-second protective layer 422, the second-third protective layer 423, and the second-fourth protective layer 424 are disposed on the first region 1A. In addition, the second-first protective layer 421, the second-second protective layer 422, and the second-third protective layer 423 are disposed on the second region 2A. That is, the second-fourth protective layer 424 is not disposed on the second region 2A.

Accordingly, a thickness of the protective layer on the second region 2A is reduced. Therefore, electromagnetic energy reception efficiency of the second pattern electrode may be improved.

A neural stimulation device according to an embodiment includes a first protective layer and a second protective layer. The second protective layer is formed of a plurality of layers.

The second protective layer includes the second-first protective layer, the second-second protective layer, the second-third protective layer, and the second-fourth protective layer.

The second-first protective layer, the second-second protective layer, and the second-third protective layer are disposed to correspond to a thickness of a molding part protecting the electronic component. In addition, the second-first protective layer, the second-second protective layer, and the second-third protective layer contact the molding part. Therefore, it is possible to prevent moisture from being introduced into the electronic component.

In addition, even when a crack or peeling of the molding part occurs as the neural stimulation device is bent, the electronic component may be protected by the second protective layer.

In addition, the second-fourth protective layer is disposed on the second-third protective layer and on an upper surface of the molding part. Accordingly, the second protective layer is planarized.

In addition, the second-first protective layer, the second-second protective layer, and the second-third protective layer may be disposed while having a step. Accordingly, a movement path of moisture is lengthened. Therefore, it is possible to prevent moisture from being introduced into the electronic component or into an interior of the neural stimulation device.

In addition, the second surface on which the electronic component is disposed may include a first region and a second region. The first region overlaps the mounting region. The second region does not overlap the mounting region. The second-first protective layer, the second-second protective layer, the second-third protective layer, and the second-fourth protective layer are disposed on the first region. At least one protective layer among the second-second protective layer, the second-third protective layer, and the second-fourth protective layer is not disposed on the second region.

Accordingly, a thickness of the protective layer on the second region is reduced. Accordingly, reception efficiency of the second pattern electrode may be improved.

The characteristics, structures and effects described in the embodiments above are included in at least one embodiment but are not limited to one embodiment. Furthermore, the characteristics, structures, and effects and the like illustrated in each of the embodiments may be combined or modified even with respect to other embodiments by those of ordinary skill in the art to which the embodiments pertain. Thus, it should be construed that contents related to such a combination and such a modification are included in the scope of the embodiment.

The description has been focused on the embodiment, but it is merely illustrative and does not limit the embodiment. A person skilled in the art to which the embodiment pertains may appreciate that various modifications and applications not illustrated above are possible without departing from the essential features of the embodiment. For example, each component particularly represented in the embodiment may be modified and implemented. In addition, it should be construed that differences related to such changes and applications are included in the scope of the embodiment defined in the appended claims.

## Claims

1. A neural stimulation device comprising:
a substrate including a first surface and a second surface opposite to the first surface;
a first pattern electrode disposed on the first surface;
a second pattern electrode disposed on the second surface;
a first protective layer disposed on the first pattern electrode; and
a second protective layer disposed on the second pattern electrode,
wherein the first pattern electrode includes a plurality of branch electrodes, a merge electrode connecting the plurality of branch electrodes, and a protrusion protruding from the branch electrode or the merge electrode,
wherein the protrusion includes at least one of a first protrusion connected to the branch electrode and a second protrusion connected to the merge electrode, and
wherein a first spacing between a branch electrode adjacent to the first protrusion and the first protrusion is 5% to 45% of a second spacing between the branch electrodes.

2. The neural stimulation device of claim 1, wherein the substrate includes a mounting region in which an electronic component is disposed, and
wherein the mounting region does not overlap the first pattern electrode in a thickness direction of the substrate.

3. The neural stimulation device of claim 1, wherein a line width of the branch electrode is 250 µm to 330 µm,
wherein a length of the protrusion is 10 µm to 50 µm, and
wherein the second spacing is 60 µm to 110 µm.

4. The neural stimulation device of claim 1, wherein sizes of the first protrusion and the second protrusion are different.

5. The neural stimulation device of claim 4, wherein the substrate includes a mounting region in which an electronic component is disposed,
wherein the mounting region does not overlap the branch electrode in a thickness direction of the substrate, and
wherein the mounting region overlaps the merge electrode in the thickness direction of the substrate.

6. The neural stimulation device of claim 4, wherein a length of the second protrusion is longer than a length of the first protrusion.

7. The neural stimulation device of claim 4, wherein a length of the second protrusion is 50% to 100% of the second spacing.

8. The neural stimulation device of claim 4, wherein the first protrusion overlaps the second protective layer in a thickness direction of the substrate, and
wherein the second protrusion does not overlap the second protective layer in the thickness direction of the substrate.

9. The neural stimulation device of claim 1, wherein the second pattern electrode is disposed in a closed-loop shape.

10. The neural stimulation device of claim 1, further comprising:
a plurality of vias penetrating the first surface and the second surface of the substrate,
wherein the first pattern electrode and the second pattern electrode are connected by the vias,
wherein the first pattern electrode includes a first metal layer and a second metal layer,
wherein the first metal layer and the second metal layer are disposed on an inner surface and a lower surface of the via, and
wherein the first protective layer is disposed inside the via.
